# EUROPEAN PATENT SPECIFICATION

(11) **EP 2 385 135 B1**
(45) Date of publication and mention of the grant of the patent: **09.09.2015**
(21) Application number: 10159524.7
(22) Date of filing: 09.04.2010
(51) Int. Cl.: C12Q 1/68

(54) **RAD51C as a human cancer susceptibility gene**
RAD51C als menschliches Krebssuszeptibilitätsgen
RAD51C en tant que gène de susceptibilité au cancer humain

(43) Date of publication of application: 09.11.2011
(73) Proprietor: Hanenberg, Helmut, 47804 Krefeld (DE); Universität zu Köln, 50923 Köln (DE)
(72) Inventor: Hanenberg, Helmut, 47804 Krefeld (DE); Schmutzler, Rita, 50931 Köln (DE); Meindl, Alfons, 81675 München (DE); Wiek, Constanze, 40591 Düsseldorf (DE); Erven, Verena, 40221 Düsseldorf (DE); Freund, Marcel, 40233 Düsseldorf (DE)
(74) Representative: PATERIS Patentanwälte PartmbB

(56) References cited:
- WO-A2-01/55450
- BARLUND MAARIT ET AL: "Multiple genes at 17q23 undergo amplification and overexpression in breast cancer" CANCER RESEARCH, vol. 60, no. 19, 1 October 2000 (2000-10-01), pages 5340-5344, XP001096150 ISSN: 0008-5472
- POOLEY KAREN A ET AL: "Common Single-Nucleotide Polymorphisms in DNA Double-Strand Break Repair Genes and Breast Cancer Risk" CANCER EPIDEMIOLOGY BIOMARKERS & PREVENTION, vol. 17, no. 12, December 2008 (2008-12), pages 3482-3489, XP002591920 ISSN: 1055-9965
- SELLICK G ET AL: "Germline mutations in RAD51, RAD51AP1, RAD51B, RAD51C, RAD51D, RAD52 and RAD54L do not contribute to familial chronic lymphocytic leukemia" LEUKEMIA AND LYMPHOMA, vol. 49, no. 1, January 2008 (2008-01), pages 130-133, XP008124183 DOI: 10.1080/10428190701606800
- GODTHELP BARBARA C ET AL: "Mammalian Rad51C contributes to DNA cross-link resistance, sister chromatid cohesion and genomic stability" NUCLEIC ACIDS RESEARCH, vol. 30, no. 10, 15 May 2002 (2002-05-15), pages 2172-2182, XP002591922 ISSN: 0305-1048
- KUZNETSOV SERGEY G ET AL: "Loss of Rad51c Leads to Embryonic Lethality and Modulation of Trp53-Dependent Tumorigenesis in Mice" CANCER RESEARCH, vol. 69, no. 3, February 2009 (2009-02), pages 863-872, XP002591923 ISSN: 0008-5472
- THACKER J: "The RAD51 gene family, genetic instability and cancer" CANCER LETTERS, vol. 219, no. 2, 10 March 2005 (2005-03-10), pages 125-135, XP004758819 NEW YORK, NY, US ISSN: 0304-3835 DOI: 10.1016/J.CANLET.2004.08.018
- MEINDL ALFONS ET AL: "Germline mutations in breast and ovarian cancer pedigrees establish RAD51C as a human cancer susceptibility gene" NATURE GENETICS, vol. 42, no. 5, May 2010 (2010-05), pages 410-416, XP002591924 ISSN: 1061-4036

## Description

### Field of the invention

The disclosure relates to *in vitro* methods for determining a genetic predisposition of a subject for developing a cancer and for assessing the pathological progression of a cancer. The invention relates to an *in vitro* method for assessing the functionality of a RAD51C gene. The invention also relates to the use of a kit for determining a predisposition of a subject for developing a cancer, and certain uses of oligonucleotides for determining the presence of at least one mono-allelic germ-line mutation of the *RAD51C* gene.

### Background of the invention

Besides most female cancers, i.e. breast and ovarian cancer, appear sporadically, 5 % to 15 % are related to an inherited susceptibility, due to alterations in the genetic code present in the familial pedigree. Several genes have been identified to be involved in hereditary breast and ovarian cancer, e.g. *BRCA1* and *BRCA2,* mostly found in families showing the Hereditary Breast and Ovarian cancer Syndrome, and *PTEN,* also found in both hereditary breast and ovarian cancer. In addition, mutations of genes linked to mismatch repair (MMR) have been observed in hereditary ovarian cancers. Another gene, *TP53,* involved in cell cycle control was found to be frequently altered in patients suffering from breast cancers associated with the Li-Fraumeni syndrome. In general, although mutations in these genes increase susceptibility to develop gynecologic cancers significantly, the mechanisms leading to tissue transformation are believed to involve the accumulation of genetic alterations, in particular in proto-oncogenes, tumor surpressor genes and mutator genes.

Despite the rising knowledge about genes involved in cancer formation and genes favoring the occurrence of cancer in general and gynecological cancers in particular, only a limited amount of genetic dispositions have been found so far. For example only up to 45 % of all hereditary breast and/or ovarian cancer families can be assigned to mutations of *BRCA1* or *BRCA2.* The gynecological cancers in the remaining approximately 55 % of families are currently explained by two different models. The model *'common diseases - rare genotypes'* postulates the existence of additional risk conferring cancer genes in which a mono-allelic germ-line mutation leads to the development of breast and/or ovarian cancer. Here, ten genes have been identified so far, most of them involved in the maintainance of genomic integrity. However, the majority (excluding *BRCA1, BRCA2, PTEN, TP53*) confers only a slightly increased cancer risk to individuals with germ-line alterations. The second currently widely favored model postulates the existence of several loci in the human genome where sequence alterations are associated with a low risk for the development of breast and/or ovarian cancer. Here, through combinations of several low risk factor loci, the individual's risk to develop cancer is determined. These loci are currently identified through genome-wide association studies (GWAS). Thus, diagnostic methods and tools are required to identify genetic mutations in genes other than the currently known cancer susceptibility genes that implicate a predisposition for gynecological cancers.

WO 01/55450 A2 discloses a method for determining predisposition to breast cancer comprising detecting genomic amplification of the RAD51C gene. This amplification leads to an increased level of the RAD51C gene product.

POOLEY KAREN A ET AL ("Common Single-Nucleotide Polymorphisms in DNA Double-Strand Break Repair Genes and Breast Cancer Risk", CANCER EPIDEMIOLOGY BIOMARKERS & PREVENTION, vol. 17, no. 12, December 2008, pages 3482-3489) discloses a monoallelic germline SNP in RAD51C which is associated with increased risk of death in breast cancer patients, but it states that none of the SNPs in RAD51C tested showed association with breast cancer predisposition.

KUZNETSOV SERGEY G ET AL ("Loss of Rad51c Leads to Embryonic Lethality and Modulation of Trp53-Dependent Tumorigenesis in Mice", CANCER RESEARCH, vol. 69, no. 3, February 2009 (2009-02), pages 863-872) discloses that RAD51C functions as a tumor suppressor in mice. However, it also states that heterozygosity for RAD51C does not predispose mice to cancer.

### Summary

The present invention relates to the embodiments as disclosed in the claims. The present invention relates to an *in vitro* method for determining a predisposition of a subject for developing breast or ovarian cancer comprising the step of analyzing *in vitro* a sample of the subject for an alteration of at least one allele of the RAD51C gene, wherein the alteration of the RAD51C gene leads to an alteration of the RAD51C gene product with reduced or abolished functionality and indicates a predisposition for developing the cancer. Further, the present invention relates to an *in vitro* method for assessing a pathological progression of a breast or ovarian cancer comprising the step of analyzing *in vitro* a sample of the cancer for a mono-allelic germ-line mutation of the RAD51C gene, wherein the presence of at least one mono-allelic germ-line mutation in the RAD51C gene leads to an alteration of the RAD51C gene product with reduced or abolished functionality and indicates an increased probability for malignancy and/or invasiveness. Furthermore, the invention provides an *in vitro* method for assessing the functionality of a RAD51C gene product, which is derived from a RAD51C gene comprising at least one alteration, in a cell derived from a subject suffering from breast or ovarian cancer, comprising the steps of
(i) introducing the RAD51C gene into the cell,
(ii) analyzing at least one cellular function, and
(iii) comparing the at least one cellular function to a control cell,
wherein a difference between the cellular function of the cell and the control cell indicates an altered function of the RAD51C gene product and wherein reduced or abolished functionality of the RAD51C gene product indicates a predisposition of the subject for developing breast or ovarian cancer.

In addition, the invention relates to the use of a kit for determining a predisposition of a subject for developing a cancer by determining the presence of at least one mono-allelic germ-line mutation of the RAD51C gene, wherein the presence of at least one mono-allelic germ-line mutation in the RAD51C gene leads to an alteration of the RAD51C gene product with reduced or abolished functionality and indicates a predisposition for developing cancer, said kit comprising oligonucleotides capable of determining the presence of at least one mono-allelic germ-line mutation of the RAD51C gene. Also provided by the present invention is the use of oligonucleotides capable of determining the presence of at least one mono-allelic germ-line mutation of the RAD51C gene in a sample of a subject leading to a RAD51C gene product with reduced or abolished functionality for determining a predisposition of the subject for developing breast or ovarian cancer.

There is also disclosed an *in vitro* method for determining a predisposition of a subject for developing a cancer comprising the step of analyzing *in vitro* a sample of the subject for an alteration of at least one allele of the *RAD51C* gene, wherein the alteration of the *RAD51C* gene leads to an alteration of the RAD51C gene product and indicates a predisposition for developing a cancer.

There is also disclosed an *in vitro* method for assessing a pathological progression of a cancer comprising the step of analyzing *in vitro* a sample of the cancer for a mono-allelic germ-line mutation of the RAD51C gene, wherein the presence of at least one mono-allelic germ-line mutation in the RAD51C gene indicates an increased probability for malignancy and/or invasiveness.

Further, there is described an *in vitro* method for assessing the functionality of a RAD51C gene product, which is derived from a RAD51C gene comprising at least one alteration, in a cell, comprising the steps of
(i) introducing the RAD51C gene into the cell,
(ii) analyzing at least one cellular function, and
(iii) comparing the at least one cellular function to a control cell,
wherein a difference between the cellular function of the cell and the control cell indicates an altered function of the RAD51C gene product.

Furthermore, there is described a kit for determining a predisposition of a subject for developing a cancer comprising oligonucleotides capable of determining the presence of at least one mono-allelic germ-line mutation of the RAD51C gene, wherein the presence of at least one mono-allelic germ-line mutation in the RAD51C gene indicates a predisposition for developing cancer.

In addition, there is disclosed the use of oligonucleotides capable of determining the presence of at least one mono-allelic germ-line mutation of the RAD51C gene in a sample of a subject for determining a predisposition of the subject for developing cancer.

The invention will be apparent to the person skilled in the art by the following description of the drawings and the detailed description of the invention.

### Description of the drawings

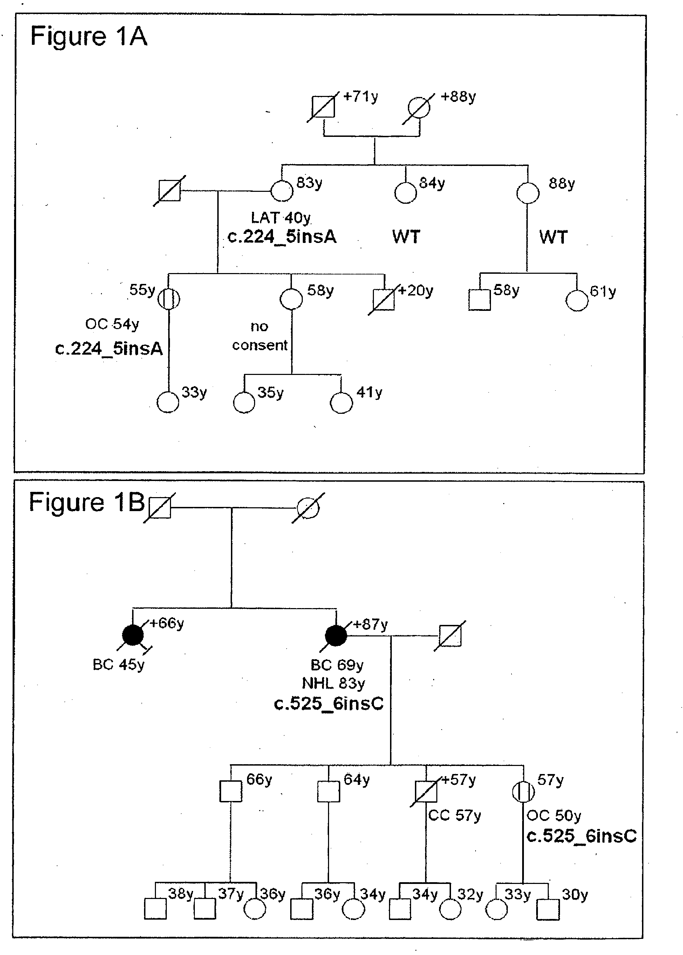

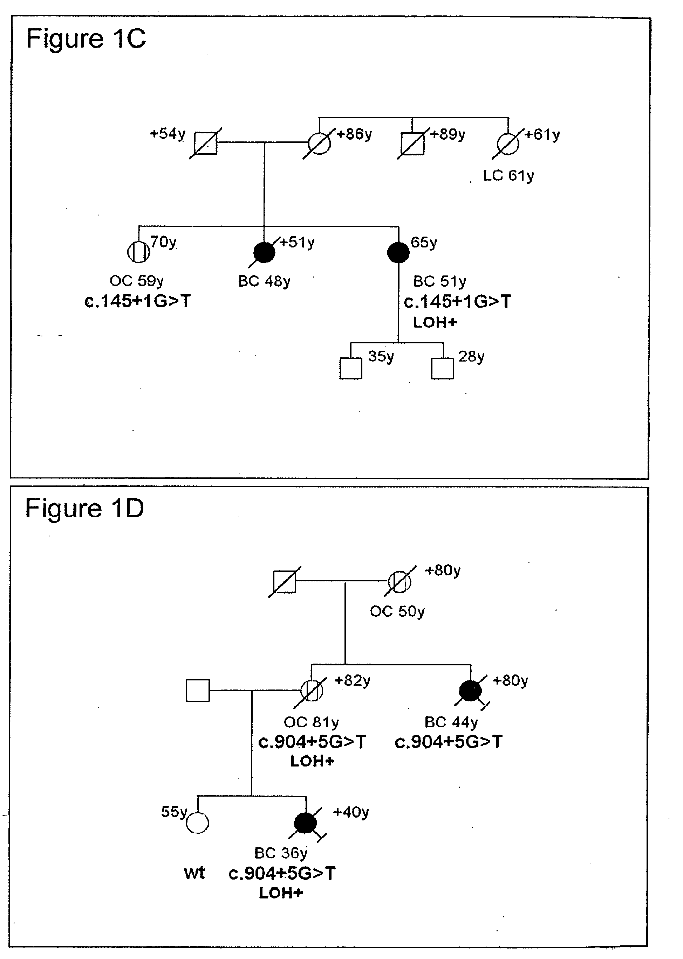

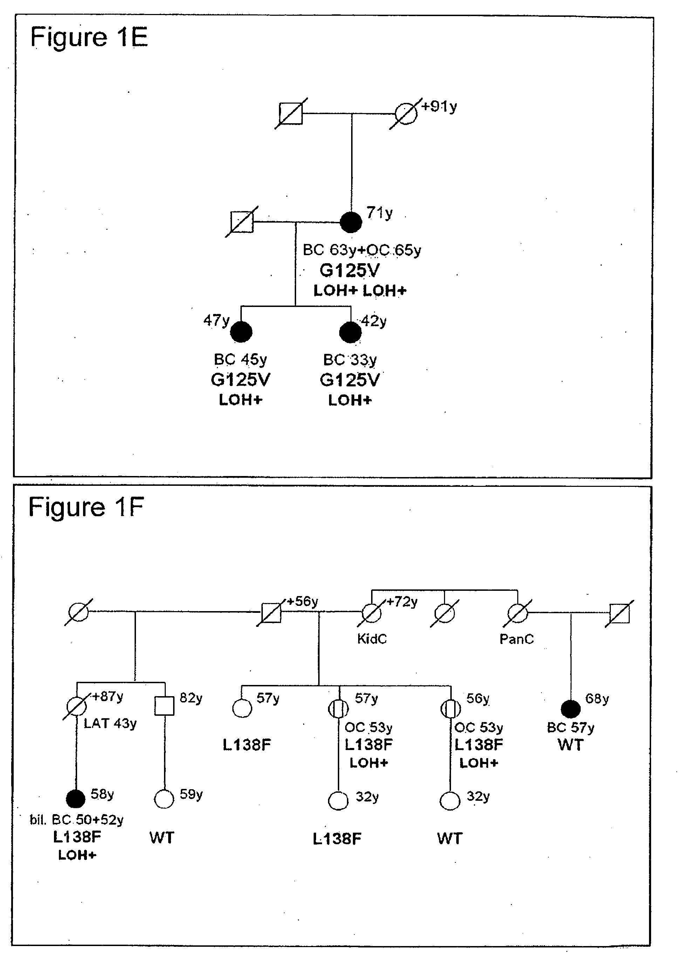

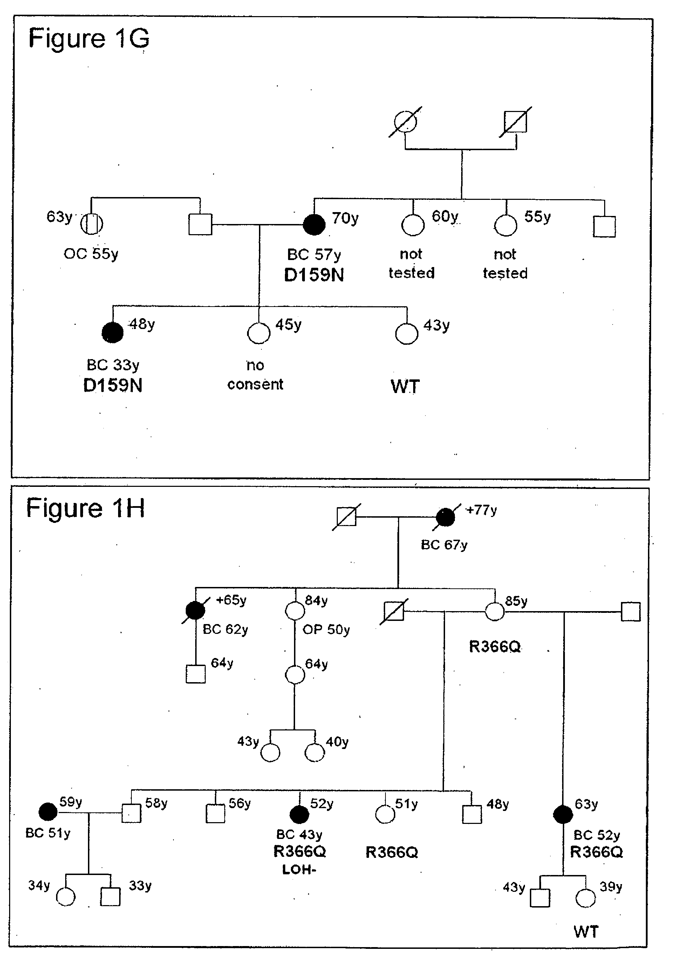

Figure 1 shows familial breast/ovarian cancer pedigrees harboring *RAD51C* mutations. Carriers of *RAD51C* mutations are shown with their specific *RAD51C* mutation (as listed in Table 3), whereas individuals tested negative for the mutation in the specific pedigree are depicted as wild-type (WT). Individuals with breast cancer (BC) are shown as filled, females with ovarian cancer (OC) as streaked circles. Disease and age at first diagnosis is given underneath, current age above the symbol. Other cancers diagnosed in the pedigrees are also shown (LC = lung cancer; KidC = kidney cancer, PanC = pancreatic cancer, CC = colon cancer, LAT = lower abdominal tumor, NHL = Non-Hodgkin-Lymphoma). All affected individuals with breast or ovarian cancer not tested for germ-line mutations in *RAD51C* were deceased or refused testing. Informed consent was obtained from all individuals tested, and the study was approved by local Ethics Committees. In addition, loss of heterozygosity (=LOH) data (+ for loss of the WT allele or - for a retained WT allele) is shown for the individuals where tissue samples of the tumor(s) could be analyzed.

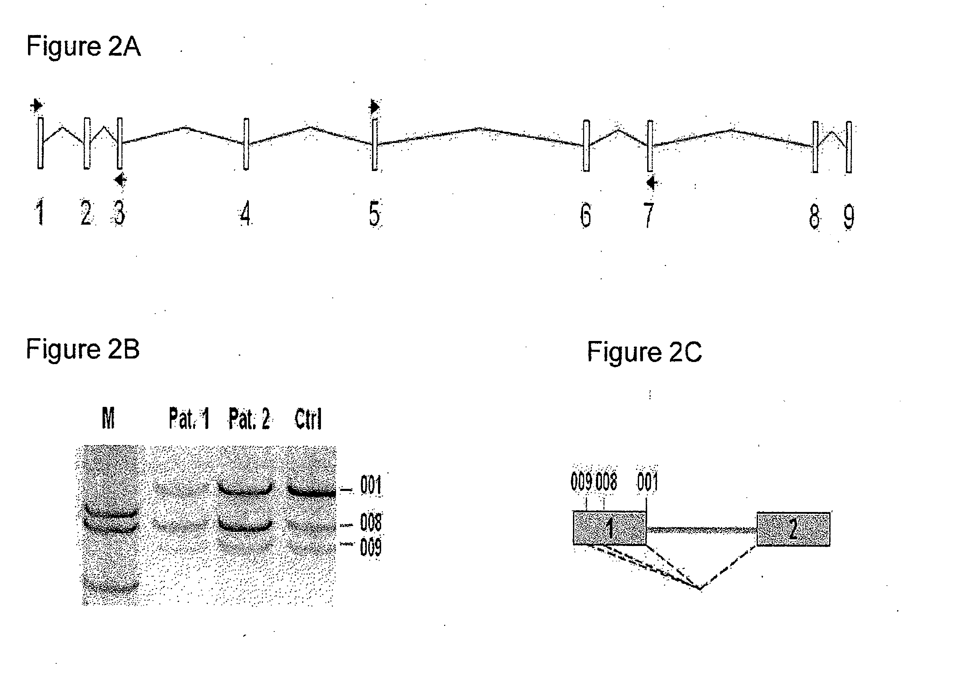

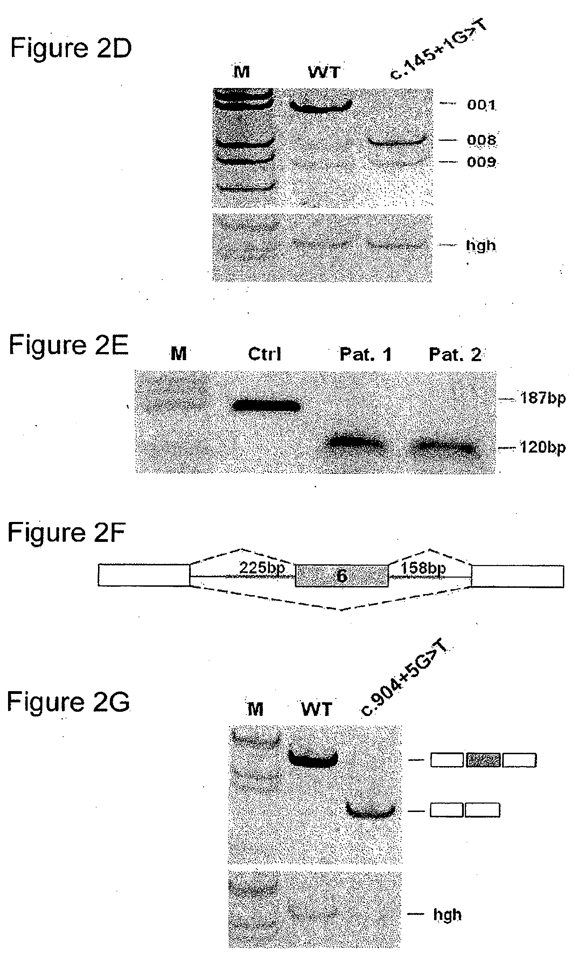

Figure 2 shows functional analyses of the splice donor mutations c. 145+1 G>T and c.904+5G>T. The RAD51C protein coding transcript 001 (OTTHUMT0000-0280540 according to http://www.ensembl.org) includes 9 exons. The primers for RT-PCR analysis are indicated by arrows (Figure 2A). Using primers located in exon 1 and exon 3, RT-PCR analysis of total RNA from PB mononuclear cells of two affected individuals with breast or ovarian cancer harboring the c.145+1G>T splice donor mutation revealed three alternative transcripts from exon 1: RAD51C-001, -008 and -009 (Figure 2B). Schematic drawing of the splice donor sites (GT) in *RAD51C* exon 1 used in the transcripts RAD51C-001, -008 and -009, respectively (Figure 2C). RT-PCR analysis of HeLa cells transfected with the RAD51C minigene splicing constructs carrying either the wild-type or the c.145+1G>T mutant 5' splice site within the RAD51C subgenomic region (Figure 2D). RT-PCR analysis of total RNA extracted from paraffin-embedded tumor samples from two carriers of the c.905+5G>T mutation in this pedigree (Figure 2E). Schematic drawing of a 3-exon splicing reporter containing the *RAD51C* exon 6 with adjacent 225 and 158 bp intronic sequences (Figure 2F). RT-PCR analysis of RNA from HeLA cells transfected with minigene constructs carrying either the WT or the c.905+5G>T mutant 5' splice site (Figure 2G).

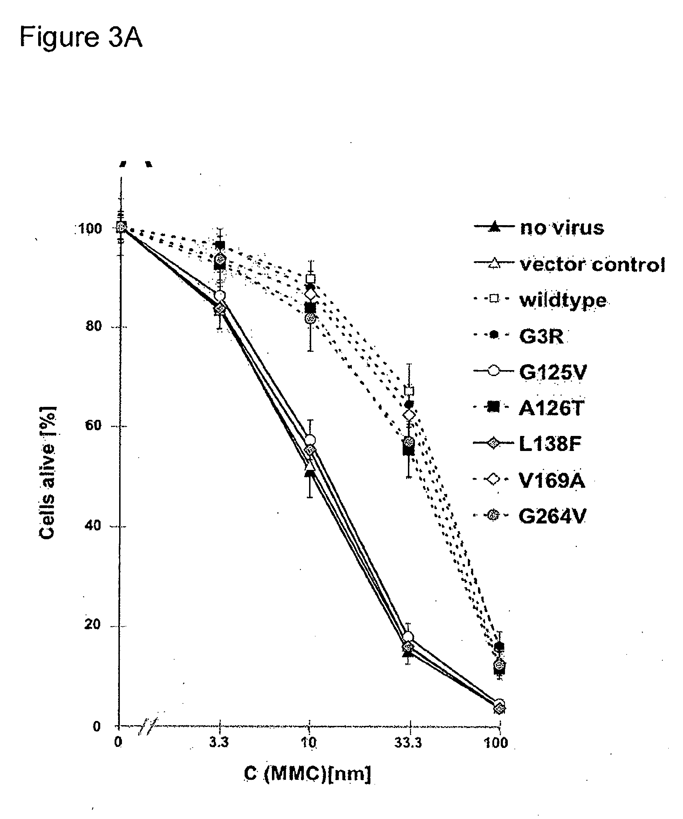

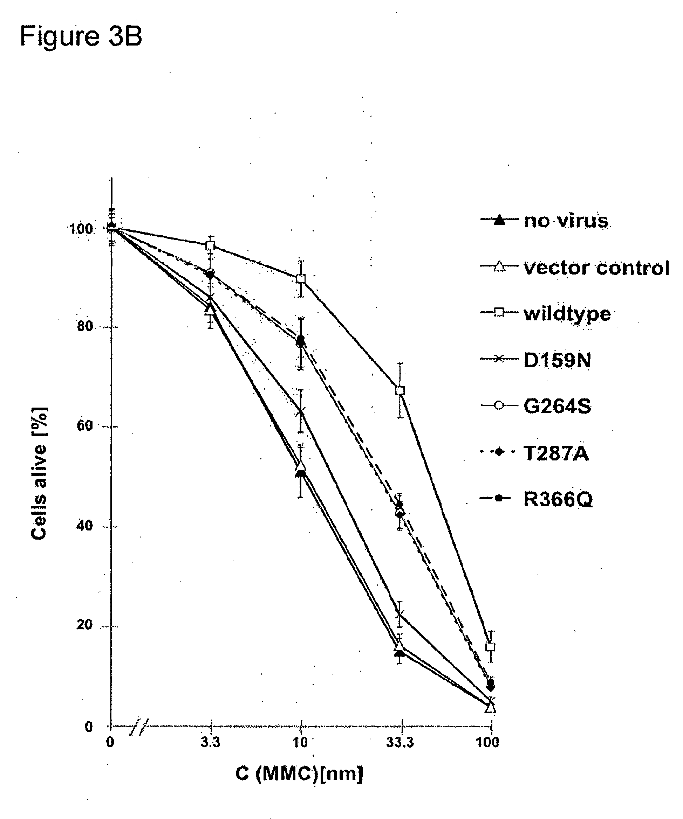

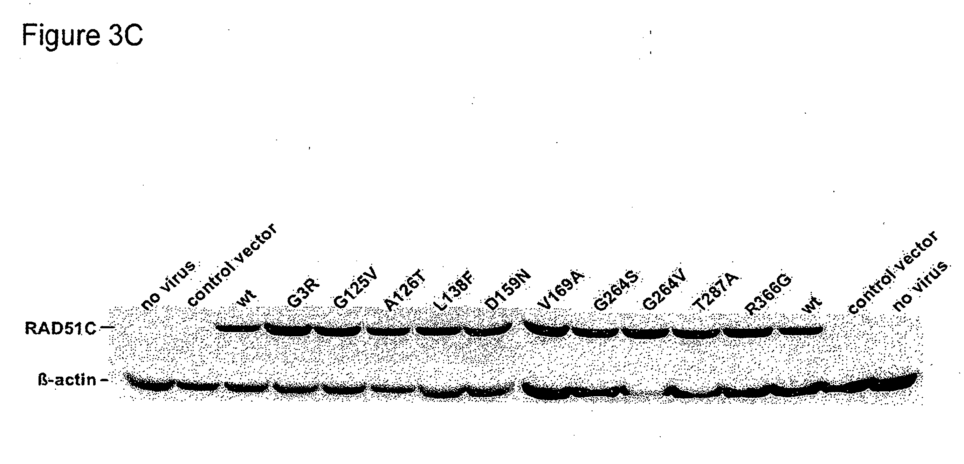

Figure 3 shows functional analyses of RAD51C missense mutations in *Rad51C* deficient DT40 cells. Analyzing the survival of *Rad51C* deficient DT40 cells transduced with mutant RAD51C proteins allows to distinguish between *Rad51C* alterations with normal function (G3R, A126T, V169A, G264V) (Figure 3A), non-functional true null-mutations (G125V, L138F) (Figure 3A) and proteins with intermediate activity (D159N, G264S, T287A, R366Q) (Figure 3B). Data are given in mean ± SD, n = 4. Western blot analysis of puromycin resistant *Rad51C*-deficient DT40 cells expressing wild-type or missense proteins from the retroviral LTR promother demonstrates equal expression of all mutant proteins (Figure 3C).

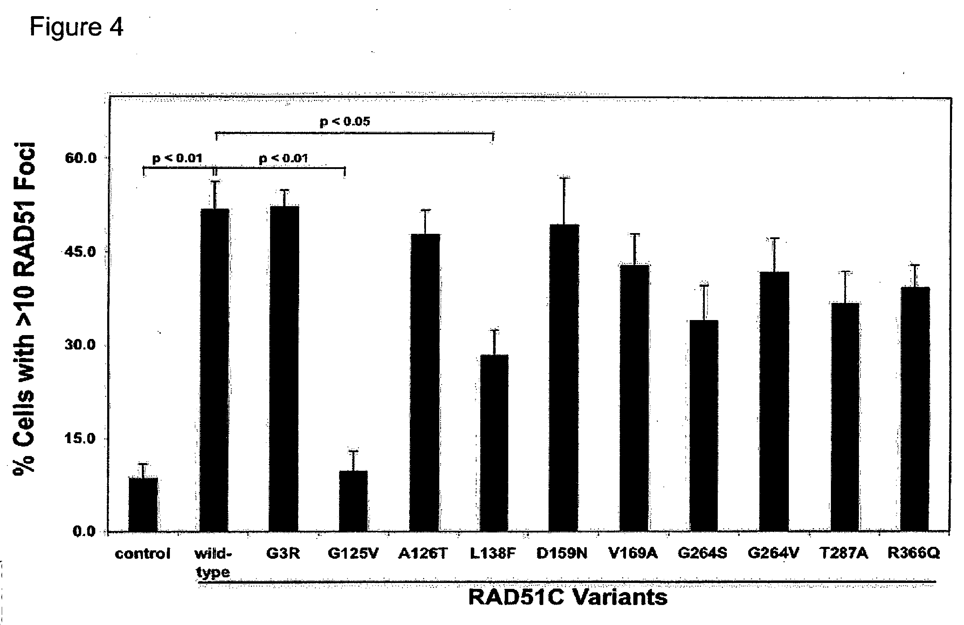

Figure 4 shows RAD51 foci formation, analyzed by immunofluorescent antibody staining, in human *RAD51C*-mutated fibroblasts transduced with retroviral vectors that expressed the ten *RAD51C* missense alterations or the wild-type *RAD51C* cDNA. Data are given in % of cells (mean ± SEM, n= 4).

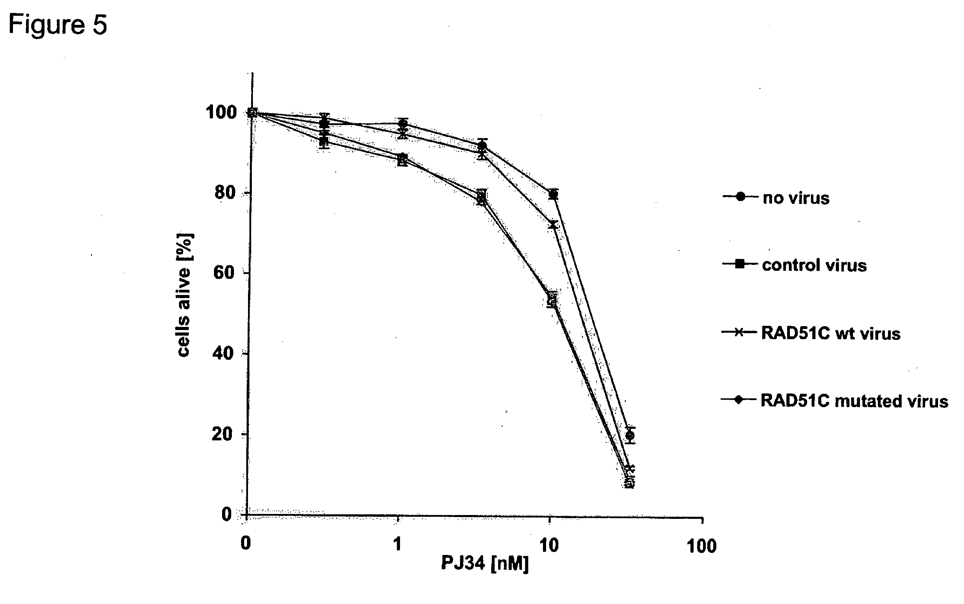

Figure 5 shows the hypersensitivity of cells with reduced or absent RAD51C function towards exposure to the representative PARP inhibitor PJ34. This increased toxicity of PJ34 is known to be specific for cells with bi-allelic defects in the *BRCA1* or *BRCA2* genes, but not FANCA or other FA deficient cells. Also *RAD51C* bi-allelic mutated cells show increased toxicities at doses leading to DNA damage that can be readily repaired by normal cells, by cells with only a mono-allelic RAD51C germ-line mutation and one wild-type RAD51C allele, and also by *RAD51C* bi-allelic mutated cells that were complemented by expression of normal RAD51C protein (means ± SD, n=3).

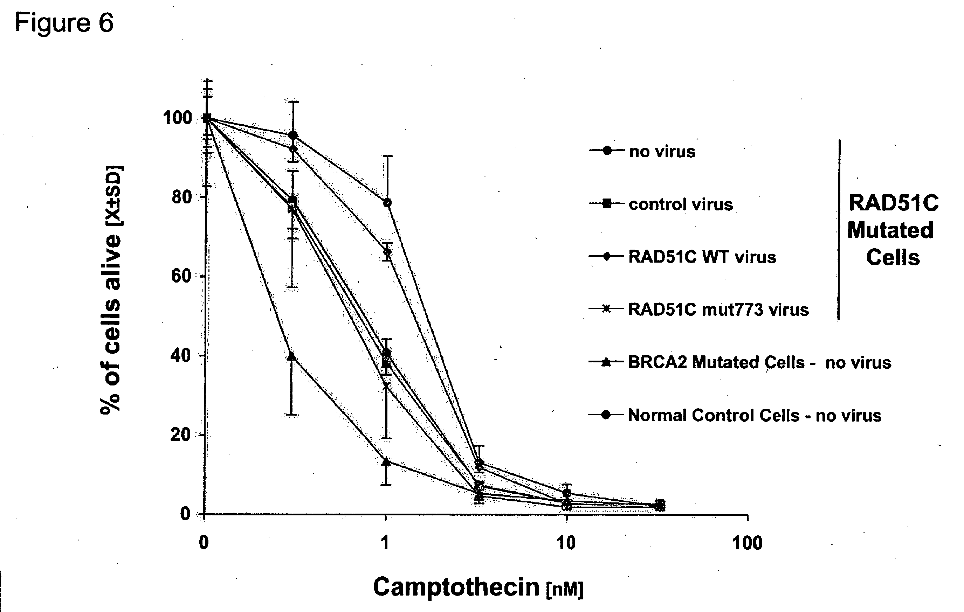

Figure 6 shows the hypersensitivity of cells with reduced or absent RAD51C function towards exposure to the representative topoisomerase I inhibitor camptothecin. This increased toxicity of camptothecin is known for FA cells with bi-allelic defects in the BRCA2 and PALB2 genes, but not in FANCA and other FA genes. Also *RAD51C* bi-allelic mutated cells show increased toxicities at doses leading to DNA damage that can be readily repaired by normal cells, by cells with only a mono-allelic RAD51C germ-line mutation and one wild-type RAD51C allele, and also by *RAD51C* bi-allelic mutated cells that were complemented by expression of normal RAD51C protein (means ± SD, n=4).

### Detailed description of the invention

1. There is described an *in vitro* method for determining a predisposition of a subject for developing a cancer comprising the step of analyzing *in vitro* a sample of the subject for an alteration of at least one allele of the RAD51C gene, wherein the alteration of the RAD51C gene leads to an alteration of the RAD51C gene product and indicates a predisposition for developing a cancer.
   The term "sample" as used herein refers to any specimen of liquids or tissue derived from a subject. The specimen comprises cells and/or nucleic acid, in particular DNA. More specifically, it refers to specimens comprising blood, epithelial cells or tumor tissue. The term "alteration" as used herein refers to any alteration of the sequence of the nucleic acid, in particular the DNA of a gene. It comprises any mutation of the nucleic acid, in particular any insertion, deletion, exchange and/or modification of the DNA molecule, in particular the nucleobases.
   To identify new genes associated with an increased predisposition of developing cancer, the inventors analyzed female index patients, from 1100 unrelated pedigrees, suffering from breast and/or ovarian cancer. All patients were selected from pedigrees with gynecological cancers to particularly search for genetic mutations which inheritably determine a susceptibility of gynecological cancers. In addition, the patients were all selected from pedigrees negative for mutations of BRCA1 and BRCA2, both genes are well known to be associated with hereditary breast and/or ovarian and also other cancers, to particularly identify genetic mutations causing a cancer predisposition independently of already known determinants. Following this approach, the gene *RAD51C* was identified to be mutated in 480 of the analyzed pedigrees, but not in 2912 healthy control subjects. All analyzed mutations were identified as mono-allelic germ-line mutations. RAD51C, Homo sapiens RAD51 homolog C, also designated RAD51L2 or MGC104277, is a member of the RAD51 family, which encode proteins involved in the repair of damaged DNA. The RAD51C protein interacts with two other DNA repair proteins, *RAD51B* and *XRCC3,* with which it forms at least two different endogenous complexes. The occurrence of specifically mono-allelic germ-line mutations of *RAD51C* is consistent with the familial accumulation of cancers in these backgrounds. Furthermore it strongly suggests that already the inheritance of a single mutated allele is sufficient to dramatically increase the likelihood of affected individuals (>80%) for developing RAD51C associated cancers until the age of 70 years. Thus, the presence of a mono-allelic germ-line mutation of RAD51C indicates a predisposition of the subject for developing cancer and establishes *RAD51C* as a high-risk cancer susceptibility gene similarly to *BRCA1* and *BRCA2.*
   The cancer may be selected from the group consisting of head and neck cancer, lung cancer, kidney cancer, pancreatic cancer, colon cancer, lower abdominal tumor, non-Hodgkin lymphoma, and gynecological cancer, in particular breast and ovarian cancer.
   The term "gynecological cancer" as used herein refers to human cancer, in particular female, gynecological tissues, as e.g. breast, ovary, cervix and uterus. It particularly comprises ductal carcinoma *in situ,* lobular carcinoma *in situ,* invasive ductual carcinoma, invasive lobular carcinoma, inflammatory breast cancer, ovarian epithelial cancer and ovarian germ cell tumors. As evident from the investigated pedigrees, RAD51C alterations are in particular associated with breast and ovarian cancer. However, also other cancers as e.g. kidney, pancreatic or colon cancer occur frequently in families showing RAD51C alterations.
   The alteration of the *RAD51C* gene may be a mutation of the *RAD51C* gene, preferably a germ-line mutation, further preferred a mono-allelic germ-line mutation. The mutation of the *RAD51C* gene may be a non-functional mutation or a loss of a *RAD51C* wild-type allele, preferably the loss of both *RAD51C* wild-type alleles.
   Mutations of the *RAD51C* gene comprise changes of the DNA sequence including insertions, exchanges or deletions of one or more nucleotides, leading to the production of a *RAD51C* gene product, i.e. RAD51C protein, with reduced or abolished functionality. Likewise, *RAD51C* mutations can cause the total absence of any RAD51C protein if transcription or translation of the *RAD51C* gene are abolished or prematurely terminated due to the mutation. An entire absence of RAD51C protein also results if both *RAD51C* wild-type alleles are lost. Cells carrying such mutations of the *RAD51C* gene lack sufficient amounts of functional RAD51C protein to maintain the required efficiency of the cellular DNA repair system. As a consequence damages and mutations accumulate within the DNA of the cell, predominantly in tumor suppressor genes causing an increased risk for cancer development.
   In a preferred embodiment the alteration of the RAD51C gene is a splice site alteration, preferably c.145+1G>T and/or c.904+5G>T, a missense alteration, preferably c.475 G>A, c.1097 G>A, c.374 G>T and/or c.414 G>C, more preferred c.374 G>T and/or c.414 G>C and/or an insertion alteration, preferably c.224_225insA and/or c.525_526insC.
   In total, fourteen mono-allelic germ-line mutations of RAD51C were detected in the 1100 pedigrees analyzed, of which eight revealed to cause functional deficits. Two mutations, c145+1G>T and c.904+5G>T, were located in splicing sites of exon 1 and exon 6 of RAD51C, respectively. The loss of the splicing site in exon 1 led to the expression of two non-functional RAD51C transcripts (RAD51C-008 and RAD51C-009) while the expression of the wild-type transcript (RAD51C-001) from this allele was lost. The second splice site mutation caused the exclusion of exon 6 from the RAD51C transcript. In addition, four missense mutations with reduced functions, c.475 G>A, c.1097 G>A, c.374 G>T and c.414 G>C, and two insertion mutations disrupting the RAD51C open reading frame, c.224_225insA and/or c.525_526insC, were found. The missense and insertion mutations all cause a change in the respective codon.
   In a preferred embodiment, the alteration of the RAD51C gene product is an alteration of the codon encoding an amino acid residue of the RAD51C protein. The identified missense mutations lead to an altered sequence of the respective codon, such that the codon encodes a different amino acid compared to the wild-type cDNA. This leads to RAD51C proteins with an amino acid sequence which differs from wild-type RAD51C in at least one amino acid. However, despite the rather limited changes in the amino acid sequence, six of ten altered RAD51C proteins showed reduced functions (G125V, L138F, D159N, G264S. T287A, R366Q) when analyzed in rescue experiments with *Rad51C* deficient chicken DT40 cells. Four out of the six mutant proteins (D159N, G264S. T287A, R366Q) showed residual RAD51C activity and two mutant proteins had no RAD51C activity at all (G125V, L138F), when compared to the nontransduced or control virus (=mock)-transduced cells. In contrast to wild-type RAD51C proteins, none of the six mutant RAD51C proteins could restore the mitomycin C sensitivity of the ΔRAD51C DT40 cells to normal levels (Figure 3A, 3B). The insertion mutations lead to a frameshift which subsequently leads to the premature termination of the translation. Thus, from RAD51C alleles harboring a c.224_225insA and/or c.525_526insC insertion mutation a functional protein can not be translated.
   In a further preferred embodiment, the alteration of the codon is selected from the group consisting of Y75XfsX0, C176LfsX26, V15KfsX9, V280GfsX11, G125V, L138F, D159N and R366Q. The functionality of RAD51C gene products carrying any of these mutations was particularly impaired, suggesting that cells comprising at least one such allele of RAD51C possess a weak DNA damage repair system. Such cells are likely to develop into cancer cells.
   The method may further comprise the step of determining the expression ratio of the RAD51C splice variants RAD51C-001 and RAD51C-008. Alternative splicing has been observed for RAD51C, with three variants encoding different isoforms, RAD51C-001, RAD51C-008 and RAD51C-009, of which the latter two are non-functional. The mutation c.145+1G>T leads to the loss of a conserved splice site donor in exon 1, such that the isoform RAD51C-001 can not be transcribed from the mutant allele. In addition, further analysis of the c.145+1G>T allele revealed reduced expression of the normal RAD51C-001 and increased expression of the non-functional RAD51C-008 transcript, while levels of the RAD51C-009 transcript were unchanged. Thus analyzing the relative expression levels of RAD51C-001 and RAD51C-008 may be used to further confirm the presence of a c.145+1G>T in the RAD51C allele of a subject.
2. There is also described an *in vitro* method for assessing a pathological progression of a cancer comprising the step of analyzing *in vitro* a sample of the cancer for a mono-allelic germ-line mutation of the RAD51C gene, wherein the presence of at least one mono-allelic germ-line mutation in the RAD51C gene indicates an increased probability for malignancy and/or invasiveness.
   The term "pathological progression of a cancer" as used herein refers to the state and condition of a cancer, in particular of a tumor tissue. The term comprises the cellular, biochemical and in particular genetic properties or features of cancer cells which are commonly used to characterize a cancer or tumor, including protein expression levels and distributions, the invasiveness and spreading of a tumor. The decreased or absent expression of the RAD51C protein or the loss of one or both wild-type alleles of the *RAD51C* gene, e.g. a non-functional germ-line mutations and a loss of heterozygosity (LOH) of the wild-type RAD51C allele, are considered to be distinct pathological features of cancer cells. Therefore, any mono-allelic germ-line mutation and/or abnormal expression of RAD51C are indicative for certain tumor features which are useful to support prognostic assessments and/or treatment strategies/decisions. The results of the *in vitro* method of the invention may contribute to the decision whether to employ surgical procedures, prophylactical approaches or the use of drugs to selectively kill malignant cells without harming normal cells (synthetic lethality).
   The *in vitro* method may further comprise the step of analyzing *in vitro* a sample of the cancer for decreased or absent expression of the RAD51C protein or for a loss of both wild-type alleles of the RAD51C gene, wherein any abnormal expression indicates an increased probability for malignancy and/or invasiveness.
   The cancer may be selected from the group consisting of head and neck cancer, lung cancer, kidney cancer, pancreatic cancer, colon cancer, lower abdominal tumor, non-Hodgkin lymphoma, and gynecological cancer, in particular breast and ovarian cancer.
3. There is also described an *in vitro* method for assessing the functionality of a RAD51C gene product, which is derived from a RAD51C gene comprising at least one alteration, in a cell, comprising the steps of
   (i) introducing the RAD51C gene into the cell,
   (ii) analyzing at least one cellular function, and
   (iii) comparing the at least one cellular function to a control cell,
   wherein a difference between the cellular function of the cell and the control cell indicates an altered function of the RAD51C gene product.
   The term "functionality" as used herein refers to the ability of the RAD51C gene product, i.e. the resulting RAD51C protein to accomplish its normal functions within the cell. So far RAD51C is known to be involved in DNA damage repair and to interact with other DNA repair proteins, it may, however, have additional, not yet discovered functions. The term "introducing" as used herein refers to any method of bringing a RAD51C gene or constructs derived from a RAD51C gene into a cell, wherein the cell may by a wild-type cell, a RAD51C deficient or RAD51C proficient cell. In particular it refers to methods introducing DNA and/or RNA templates or constructs via transduction or transfection. DNA and/or RNA templates comprise expression vectors, viral vectors, artificial chromosomes and splicing constructs. The term "cellular function" as used herein refers to any common cellular activity including growth, proliferation, apoptosis, DNA transcription, protein expression, DNA-protein interaction and activities related to the individual cell type.
   Damages to the DNA as e.g. single strand or double strand breaks have significant effects on various cellular functions as DNA replication, cell proliferation or protein expression. Therefore, changes in the functions of the cell upon expression of a distinct RAD51C gene product, e.g. carrying a particular mutation, allows for assessing the functionality of the expressed RAD51C gene product.
   The cellular function may be selected from the group consisting of the sensitivity of the cell towards a DNA cross-linker, the cell cycle distribution, RAD51 foci formation and expression of the splice pattern of a marker construct. These cellular functions are closely related to the known functions of RAD51C in DNA repair.
   The cell may be a RAD51C deficient cell, more preferred a RAD51C deficient cell from human, hamster or chicken origin.
   In a preferred embodiment, at least one allele of the *RAD51C* gene (*RAD51C*+/-), preferably both alleles of the *RAD51C* gene (*RAD51C*-/-) is/are mutated.
   The cell may be derived from a subject suffering from a cancer selected from the group consisting of head and neck cancer, lung cancer, kidney cancer, pancreatic cancer, colon cancer, lower abdominal tumor, non-Hodgkin lymphoma, and gynecological cancer, in particular breast and ovarian cancer.
4. There is also disclosed a kit for determining a predisposition of a subject for developing a cancer comprising oligonucleotides capable of determining the presence of at least one mono-allelic germ-line mutation of the RAD51C gene, wherein the presence of at least one mono-allelic germ-line mutation in the RAD51C gene indicates a predisposition for developing cancer. With respect to the requirement of a fast and reliable analysis for medical investigations a kit provides a handy tool which may be directly used in the medical laboratory of a hospital or a physician's practice.
   The cancer may be selected from the group consisting of head and neck cancer, lung cancer, kidney cancer, pancreatic cancer, colon cancer, lower abdominal tumor, non-Hodgkin lymphoma, and gynecological cancer, in particular breast and ovarian cancer.
   The oligonucleotides may be selected from the group consisting of SEQ ID NO:11 to SEQ ID NO: 28, in particular at least one combination of SEQ ID NO:11/12, SEQ ID NO: 13/14, SEQ ID NO: 15 /16; SEQ ID NO: 17/18, SEQ ID NO: 19/20, SEQ ID NO: 21/22, SEQ ID NO: 23/24, SEQ ID NO: 25/26 and 27/28.
5. The invention is directed to the use of oligonucleotides which are capable of determining the presence of a least one mono-allelic germ-line mutation of the RAD51C gene in a sample of a subject for determining a predisposition of the subject for developing cancer.

In a preferred embodiment the oligonucleotides are selected from the group consisting of SEQ ID NO:11 to SEQ ID NO: 28, in particular at least one combination of SEQ ID NO:11/12, SEQ ID NO: 13/14, SEQ ID NO: 15 /16; SEQ ID NO: 17/18, SEQ ID NO: 19/20, SEQ ID NO: 21/22, SEQ ID NO: 23/24, SEQ ID NO: 25/26 and 27/28.

In addition, a method for treating a subject suffering from a cancer is disclosed, comprising the steps of
(i) analyzing a sample of the cancer for reduced or absent function of RAD51C,
(ii) treating the subject with an agent which inhibits DNA repair,
wherein the reduced or absent function of RAD51C leads to an increased sensitivity against the agent, such that the cancer cells are selectively killed by the agent.

The agent may be a (Poly [ADP-ribose] polymerase (PARP) or Topoisomerase I (TOPO I) inhibitor.

A new treatment concept is based on the assumption that two genes are in a synergistic lethal relationship if the loss-of-function mutation in either gene alone does not lead to cell death, but concurrent mutations in both genes are lethal (Fong et al., 2009). The authors described that tumors of patients with germ-line mutations in *BRCA1* and *BRCA2* were responsive to PARP inhibitors while normal cells of these patients or tumor cells from patients with intact *BRCA1 or BRCA2* genes were not affected. The inventors showed that primary *RAD51C* mutated cells are also hypersensitive to PARP inhibitors. In the pedigrees shown in Figures 1 A-F with non-functional germ-line RAD51C mutations, the wild-type allele was lost in all tumor tissues obtained from 12 breast or ovarian cancer patients. Without any residual expression of normal RAD51C protein, these tumor cells, but not the normal heterocygote cells in these affected individuals are hypersensitive to substances such as, but not limited to, PARP and TOPO I inhibitors. Therefore, assessing the RAD51C status in a tumor sample can be utilized to individually tailor treatment strategies for the affected individual using for example PARP or TOPO I inhibitors.

Further aspects of the invention will be apparent to the person skilled in the art by the enclosed description of the examples, in particular the scientific results.

### Examples - Material and Methods

### Patients and families/pedigrees

Index patients from 1100 German pedigrees with hereditary gynecological malignancies were recruited through a clinicogenetic counselling program at five Centers (Cologne, Dresden, Duesseldorf, Munich, Ulm) from the German Consortium of Hereditary Breast and Ovarian Cancer (GC-HBOC). 620 pedigrees fulfilled the criteria that at least three or more affected females with breast cancer but no ovarian cancers were present in the pedigrees (BC pedigrees). In 480 pedigrees, at least one case of breast and one ovarian cancer had occurred (BC/OC pedigrees). All patients in the study here were excluded from carrying pathogenic germ-line mutations in *BRCA1* and *BRCA2* by the PCR-based mutation detection techniques dHPLC and/or direct DNA-sequencing and the MLPA technique. In total, 2912 age-matched control samples from healthy women were collected in Northrhine-Westphalia or provided by KORA ("Kooperative Gesundheitsforschung in der Region Augsburg") (Arking et al., 2006). 480 samples were completely sequenced, 2432 samples were screened by the MALDI-TOF technique.

### Direct sequencing and dHPLC

For the rapid and reliable identification of *RAD51C* mutations, two different approaches were utilized. Mutation detection was performed by dHPLC (WAVE system, Transgenomic, Omaha, NE U.S.A.) or by direct DNA sequencing on ABI3100 sequencers (PE Applied Biosystems, Foster City, CA, U.S.A.). The primer pairs used for the amplification of the nine exons of the *RAD51C* gene are summarized in the following table:

**Table 1: Primer pairs for RAD51C**

| **exon** | **primer*** | **sequence 5' - 3'** | **sequence protocol** |
|---|---|---|---|
| Ex1 | 1F | tccgctttacgtctgacgtc | SEQ ID NO: 11 |
| Ex1 | 1R | aggcgagagaacgaagactg | SEQ ID NO: 12 |
| Ex2 | 2F | cactcctagcatcactgttg | SEQ ID NO: 13 |
| Ex2 | 2R | ttggtttcctgacgatagtac | SEQ ID NO: 14 |
| Ex3 | 3F | atttctgttgccttggggag | SEQ ID NO: 15 |
| Ex3 | 3R | aatggagtgttgctgaggtc | SEQ ID NO: 16 |
| Ex4 | 4F | tgccaatacatccaaacaggt | SEQ ID NO: 17 |
| Ex4 | 4R | gtaggtcaaggaaggaagag | SEQ ID NO: 18 |
| Ex5 | 5F | ttttcctgtaatggactatgg | SEQ ID NO: 19 |
| Ex5 | 5R | tgtcaggcaaacgctattttg | SEQ ID NO: 20 |
| Ex6 | 6F | tcacaatcttggccagactggtc | SEQ ID NO: 21 |
| Ex6 | 6R | aacggtactgtgcttagtgc | SEQ ID NO: 22 |
| Ex7 | 7F | ttccaggttttttgaaagcaag | SEQ ID NO: 23 |
| Ex7 | 7R | taggtgatatcagacaaggc | SEQ ID NO: 24 |
| Ex8 | 8F | catacgggtaatttgaaggg | SEQ ID NO: 25 |
| Ex8 | 8R | atgcttgctgcctacagaag | SEQ ID NO: 26 |
| Ex9 | 9F | ctggccctagaataaagtag | SEQ ID NO: 27 |
| Ex9 | 9R | ggtaacaagtccacttgtac | SEQ ID NO: 28 |

| | | | |
|---|---|---|---|
| *F=forward, R=reverse | | | |

The sequences of nine exons (Ex1 - Ex9; SEQ ID NO: 1 to SEQ ID NO: 9) of the *RAD51C* gene, which were generated by PCR, are given. The primer sequences (SEQ ID NO: 11 to SEQ ID NO: 28) are underlined, intronic sequences are in lower case letters, exon sequences are in capital letters, start codon in bold, stop codon in italic. The first primer in each sequence is the forward primer (1 F to 8F), and the second primer is the reverse primer (1R to 8R).
**Exon 1 (Ex1, SEQ ID NO:1) and primer 1F and 1R (SEQ ID NO: 11/12)**
**Exon 2 (Ex2, SEQ ID NO:2) and primer 2F and 2R (SEQ ID NO: 13/14)**
**Exon 3 (Ex3, SEQ ID NO:3) and primer 3F and 3R (SEQ ID NO: 15/16)**
**Exon 4 (Ex4, SEQ ID NO:4) and primer 4F and 4R (SEQ ID NO: 17**/**18**)
**Exon 5 (Ex5, SEQ ID NO:5) and primer 5F and 5R (SEQ ID NO: 19/20)**
**Exon 6 (Ex6, SEQ ID NO:6) and primer 6F and 6R (SEQ ID NO: 21/22)**
**Exon 7 (Ex7, SEQ ID NO:7) and primer 7F and 7R (SEQ ID NO: 23/24)**
**Exon 8 (Ex8, SEQ ID NO:8) and primer 8F and 8R (SEQ ID NO: 25/26)**
**Exon 9 (Ex9, SEQ ID NO:9) and primer 9F and 9R (SEQ ID NO: 27/28)**

Appropriate dHPLC conditions for running temperatures and buffer gradients were established for each individual exon. ABI BigDye terminator chemistry (PE Applied Biosystems) was used for cycle sequencing. All mutations detected by DHPLC were confirmed by direct DNA sequencing. The full length *RAD51C* gene sequence is indicated in SEQ ID NO: 10.

### PCR and DHPLC conditions

PCR and DHPLC conditions are summarized in Table 2.

**Table 2**

| | **Annealing-Temp** | | **PCR** | **TD 60°-57°** |
|---|---|---|---|---|
| Exon1 | 55 °C | | 1. 95° 5min | 1. 95° 5min |
| Exon2 | 55 °C | | 2. 95° 30s | 2. 95° 30s |
| Exon3 | 56 °C | | 3. x° 30s | 3.60° 30s |
| Exon4 | 56 °C | | 4. 72° 2min | 4. 72° 2min |
| Exon5 | 56 °C | | 5. go to 2, 34x | 5. go to 2, 5x |
| Exon6 | 56 °C | TD60-57 | 6. 72° 8min | 6. 95° 30s |
| Exon7 | 54 °C | | | 7. 59° 30s |
| Exon8 | 53 °C | | | 8. 72° 2min |
| Exon9 | 53 °C | TD60-53 | | 9. go to 6, 5x |
| | | | | 10. 95° 30s |
| | | | | 11. 58° 30s |
| | | | | 12. 72° 2min |
| | | | | 13. go to 10, 5x |
| | | | | 14. 95° 30s |
| | | | | 15. 57° 30s |
| | | | | 16. 72° 2min |
| | | | | 16. 72° 2 min |
| | | | | 17. go to 14, 19x |
| | | | | 18. 72° 8min |

### Sequencing of RAD51C transcripts

*RAD51C* transcripts from peripheral blood mononuclear cells were amplified by RT-PCR with primers *RAD51C* exon 1 and 3 and separated on 6% polyacrylamide gels. RT-PCR products were extracted with elution buffer (0.5 M ammonium acetate, 10 mM magnesium acetate, 1 mM EDTA, 0.1% SDS), reamplified with the RT-PCR primers using 2.5 U *Pwo* DNA polymerase (Roche Molecular Biochemicals), gel-extracted (Gel extraction kit, Qiagen) and directly sequenced.

### MALDI-TOF and Statistics

MALDI-TOF: The homogeneous mass-extension (hME) process was used for producing primer extension products analysed on a MALDI-TOF mass-spectrometer (Sequenom MassArray system). Assays were designed with the SpectroDesigner software. Statistics: P-values were calculated from standard chi-square tests based on allele counts. Odds ratios were calculated using standard methods. All calculations were done using R 2.91.

### RNA extraction

Total RNA was isolated from paraffin-embedded tumor samples by usage of RNease FFPE kit (Qiagen, Hilden, Germany) that obtained intact RNA molecules with a length of about 150 nucleotides. PAXgene Blood RNA tubes® were used for collection and stabilization of blood samples from patients and total RNA was isolated from blood samples with the PAXgene Blood RNA Kit according to the manufacturer's instructions (PreAnalytiX, Qiagen, Hilden, Germany).

### Splicing analysis

RT-PCR was performed with total RNA extracted from patient-derived blood samples. For analysis of the splicing pattern, prior to reverse transcription, 3µg of total RNA were subjected to DNase I digestion with 10 U of DNase I (Roche Molecular Biochemicals, Basel, Switzerland). 3µl of the treated RNA samples were reverse-transcribed with the SuperScript^{™} III RT-PCR System with Platinum Taq Polymerase (Invitrogen, Karlsruhe, Gemany) using primer pairs specific for *RAD51C* exon 1 and exon 3 (see above). To ensure a linear PCR amplification range allowing semiquantitative assessment of the spliced products, PCR analysis was performed with 28 cycles. PCR products were separated on 6% nondenaturing polyacrylamide gels, stained with ethidium bromide, visualized and quantified with the Lumi-Imager F1 (Roche Molecular Biochemicals), and directly sequenced after gel extraction.

### Splicing reporter assays

For testing the c.145+1G>T mutation, the subgenomic region spanning *RAD51C* exon 1, intron 1 and exon 2 was amplified from control DNA by PCR with primers as indicated in table 1 online using Expand^{™} High Fidelity DNA Polymerase (Roche Molecular Biochemicals). The PCR product was cloned into the pSVT7 expression vector and controlled by sequencing. The 5' splice site mutation was introduced by PCR mutagenesis using the *RAD51C* intron 1 forward (F) and reverse (R) mutagenesis primers with the QuickChange XL Site-Directed Mutagenesis Kit (Stratagene). For functional analysis of the c.904+5G>T mutation, the *RAD51C* exon 6 including the flanking intronic sequences was amplified from genomic DNA by PCR with Expand^{™} High Fidelity DNA Polymerase and inserted into the splicing reporter construct as described by Betz et al., 2009. The mutation was inserted by PCR mutagenesis using the RAD51C intron 6 forward (F) and reverse (R) mutagenesis primers. Total RNA was isolated 30h after transfection using GenElute^{™} Mammalian Total RNA Miniprep Kit (Sigma) and 200 ng of the DNase I (Roche Molecular Biochemicals) treated RNA was reverse-transcribed with the SuperScript^{™} III RT-PCR System with Platinum Taq Polymerase (Invitrogen) with vector specific primers.

### LOH analysis

DNA was extracted from sections of paraffin-embedded tumor tissues (QIAamp DNA FFPE tissue kit, Qiagen) after macrodissection to ensure amount of tumor cells > 80% as visualized in a HE stained control section. *RAD51C* DNA fragments were specifically amplified using appropriate primer pairs as indicated in table 1, directly sequenced by ABI sequencer 3130XL and compared to sequencing results of heterozygous germ-line DNA.

### Complementation of Rad51c deficient DT40 cells with RAD51C missense mutations

*Rad51c* deficient DT40 cells were purchased from the Riken BRC Cell Bank (Tsukuba, Ibaraki, Japan). The control vector S11IP, expressing an IRES-*pac* cassette, and the S11RCIP, additionally expressing the wild-type *RAD51C* cDNA from the same expression cassette were constructed as described by Vaz et al., (submitted). The patient-derived missense mutations in the *RAD51C* open reading frame were introduced using a Quick Mutagenesis kit (Stratagene, Amsterdam, The Netherlands). Stable oncoretroviral cell lines were generated and the nonadherent Δ*Rad51c* DT40 cells were transduced with retroviral supernatant. Transduced cells were selected in the presence of puromycin (Gibco/Invitrogen) for 4-5 days, exposed for three days to increasing concentrations of MMC and then assayed by flow cytometry for survival of cells using propidium iodide (Sigma-Aldrich, Taufkirchen, Germany) for live/dead cell discrimination.

### Protein expression of Rad51c missense mutations in ΔRad51c DT40 cells

Immunoblots were performed with samples containing 50 µg of total protein on 4-12% NuPage Bis-Tris polyacrylamide gels (Invitrogen, Karlsruhe Germany). Membranes were probed with mouse monoclonal anti-human RAD51C (1:1000; ab55728 abcam, Cambridge UK) or mouse monoclonal anti-β-Actin (1:5000; A2228 Sigma-Aldrich, Taufkirchen, Germany) antibodies. A secondary horseradish peroxidase-linked sheep-anti-mouse IgG (RPN4201 GE Healthcare, Munich, Germany) was used at a dilution of 1:10.000 and detected by the chemiluminescence technique using the ECL system (Pierce, Thermo Fisher Scientific, Bonn, Germany).

### RAD51 Foci Immunofluorescence

For indirect immunofluorescence staining of RAD51 foci, cells were seeded onto coverslips (Nalgene NUNC, Wiesbaden, Germany) and then the next day incubated with 150 nM mitomycin C (MMC, Medac, Germany). 24 hours later, cells were fixed with 3.7% paraformaldehyde (Sigma Aldrich, Taufkirchen, Germany) for 15 min at room temperature, and permeabilized with 0.5% Triton X-100 for 5 min. After 30 min in blocking buffer (10% BSA, PAA, Colbe, Germany; 0.1% NP-40, Sigma-Aldrich), cells were incubated at 4 °C with anti-RAD51 rabbit antibody (PC130, Calbiochem, Darmstadt, Germany) at 1/200 dilution for 45 min. Cells were washed three times in TBS (Invitrogen) and subsequently incubated with a 1/500 diluted TexasRed-conjugated anti-rabbit polyclonal antibody (Jackson Immunoresearch, Suffolk, UK). After 45 min, cells were washed three times with TBS and the slides were mounted in ProLong Gold antifade reagent (Invitrogen) with 4,6-diamidino-2-phenylindole (DAPI, Sigma Aldrich). Specimens were viewed with an inverted microscope (Axiovert 200M, Zeiss) and fluorescence imaging workstation. Images were acquired at room temperature with a Plan-Apochromat 63x/1.4 oil lens using a digital camera (AxioCam MRm, Zeiss). RAD51 foci were counted independently in three different experiments by two different people, blinded for each condition. Statistical analysis was performed using the SPSS software.

### Examples- results

In total, the inventors detected fourteen mono-allelic germ-line sequence alterations in *RAD51C* in 1100 unrelated female patients from hereditary BC and BC/OC pedigrees: two single base pair insertions, two splice site mutations and ten sequence alterations leading to single amino acid changes (Table 3). Extended family trees for eight sequence alterations are shown in Figure 1A-H, with all individuals depicted being at least 30 years of age.

### Insertion mutations

The two insertion mutations were c.224_225insA, predicted to cause p.Y75XfsX0, and c.525_526insC, leading to p.C176LfsX26. Both mutations lead to a frame shift during translation and subsequently to premature protein termination and were both clearly pathogenic.

### Splice donor mutations

The splice donor mutation (c.145+1 G>T) present in a family with three sisters affected by breast or ovarian cancers (Figure 1C) disrupts the canonical GT dinucleotide. Comparison of the *RAD51C* splicing pattern in peripheral blood leukocytes from two heterozygous mutation carriers (Figure 2A, 2B) revealed reduced expression of the normal RAD51C-001 and increased expression of the non-functional RAD51C-008 transcript, while levels of the non-functional RAD51C-009 transcript were unchanged compared to controls (transcripts and nomenclature according to http://www.ensembl.org). The latter two transcripts utilized alternative 5' splice sites with intrinsic strengths of 17.4 and 16.1, respectively, as predicted by the HBond algorithm for 5' splice sites and were confirmed by sequencing of the splice junctions in the different RT-PCR products. To prove that the normal *RAD51C* transcript was solely expressed from the wild-type allele in the heterozygous leukocytes, the inventors introduced exon 1, intron 1 and exon 2 of the *RAD51C* gene into a splicing construct (Figure 2C) (Betz et al., 2009). RT-PCR analysis following transfection of HeLa cells with the wild-type splicing reporter construct revealed usage of the exon 1 splice donor comparable to normal controls (Figure 2D). In contrast, the RT-PCR analysis of the c.145+1G>T splicing reporter demonstrated a complete inactivation of this mutant 5' splice site and increased transcript levels from the upstream proximal 5' splice site. Finally, the pathogenic nature of this 5' splice site mutation was further emphasized by the loss of the wild-type allele in the cancer tissue of the surviving breast cancer patient in pedigree 1C.

The second splice site mutation (c.904+5G>T) also affected an evolutionary conserved position and was predicted to dramatically decrease the complementarity between the U1 snRNA and the 5' splice site: the HBond score decreased from 15.8 to 10.1, thereby predicting aberrant splicing. As cells carrying the germ-line mutation were not available (Figure 1D), mRNA was isolated from paraffin-embedded tumor samples from two carriers of this mutation. As shown in Figure 2E, the inventors were able to specifically amplify an RT-PCR product that lacked exon 6 in these carriers, but not in control samples. For further confirmation, the inventors introduced the *RAD51C* exon 6 with flanking 225 and 158 bp intronic sequences into a splicing reporter construct (Figure 2F) (Betz et al., 2009). Functional analysis in this heterologous context also demonstrated that the c.904+5G>T mutation resulted in exclusion of exon 6, suggesting that the *RAD51C* exon 6 is recognized by exon definition (Figure 2G). Finally, sequencing of DNA extracted from paraffin-embedded samples revealed that a loss of the wild-type allele had occurred independently in the breast and the ovarian cancer tissues in two patients from pedigree D.

### Missense mutations

To initially screen whether the ten missense amino acid alterations are changing RAD51C protein function, the inventors used retroviral vectors to introduce the mutant human RAD51C proteins into chicken DT40 cells in which the *RAD51C* orthologue had been disrupted. Survival of cells expressing the two missense alterations at highly conserved amino acids G125V and L138F was identical to the survival of control vector-transduced or untransduced Δ*Rad51c* DT40 cells (Figure 3A) and therefore completely failed to complement the *Rad51c* mutant phenotype of these cells. In contrast, expression of the G3R, A126T, V169A and G264V missense *RAD51C* cDNAs corrected the mitomycin C (MMC) hypersensitivity of *Rad51c* mutant DT40 cells to levels achieved by expression of the wild-type *RAD51C* cDNA (Figure 3A). Expression of the four missense alterations D159N, G264S, T287A and R366Q only partially restored the MMC sensitivity of Δ*Rad51C* DT40 cells when compared to the wild-type cDNA (Figure 3B) indicating hypomorphic mutations with reduced protein activity.

For further functional analysis of these missense mutations in human cells, the inventors employed human *RAD51C*-mutated skin fibroblasts (Vaz et al., Nat Genet in press). Assessing RAD51 foci in these cells after crosslinker exposure revealed that expression of the two mutations G125V and L138F failed to restore normal RAD51 foci formation (Figure 4), whereas *RAD51C*-mutated cells transduced with vectors expressing the remaining eight *RAD51C* missense variants were functionally indistinguishable from cells expressing the wild-type *RAD51C* cDNA.

The amino acid change D159N was associated with reduced survival in Δ*Rad51C* DT40 cells, albeit demonstrating normal RAD51 foci formation when expressed in human *RAD51C*-mutated cells, was considered as unclassified variant (UCV) unique in this pedigree affected by breast cancer only (Figure 1G). Finally, despite of intermediate complementation in DT40 cells, predictive algorithms, and amino acid conservation in *RAD51C* homologues, the R366Q variants segregation was incomplete and the tested tumor lacked LOH (Figure 1H).

For further characterization of the two recurrent missense mutations G264S and T287A, showing reduced survival of DT40 cells and normal RAD51 foci formation, an association study was performed. Both, T287A and G264S revealed no association with cancer in 1100 index cases compared to 2912 healthy controls. However, the G264S variant was associated with malignancies in the subgroup of BC/OC families (Table 1; OR = 3.44; CI = 1.51-7.80, p = 5.32 x 10⁻³). Whether this represents a real association, requires further assessment in larger studies. Preliminary, this variant was also designated as UCV.

### Comparison to BRCA912 induced cancer

The occurrence of both, breast and ovarian cancers, and the high frequencies showed remarkable resemblance to the clinical presentations of *BRCA1* and *BRCA2* mutation carriers. It is well established that the breast cancer histology in *BRCA1* carriers demonstrates an excess of ductal tumors with high mitotic count and negativity for hormone receptors and HER2/neu, when compared to *BRCA2* mutation carriers or sporadic breast cancers. Pathology reports were available from 11 BC cases of pedigrees A-F. Ten were classified as invasive and one as preinvasive ductal carcinomas. Only three of the ten invasive cancers presented with lymph node infiltration. Three cases were high grade (G3) and eight cases were intermediate grade (G2) tumors. Hormone receptor status was available for ten tumors: five were negative and five positive for the estrogen (ER) and progesterone (PR) receptors. Of these, seven showed a concordant status, i. e. three were ER and PR negative and four were ER and PR positive. Three tumors harbored a discordant receptor status, i. e. two were ER negative and PR positive and one was ER positive and PR negative. All six breast cancers with HER2/neu status had a negative score. Of those, five were either ER or PR positive and one was negative for both receptors. These results indicate that *RAD51C*-associated breast cancer is distinct from *BRCA1*-associated breast cancer and might be associated with more favourable histopathological features such as BRCA2-associated breast cancer. Pathological reports were available for seven *RAD51C-*associated ovarian cancers, six of which presented as invasive serous and one as invasive endometrioidal adenocarciomas. Three ovarian cancers were classified as high grade (G3) and four as intermediate grade (G2) tumors. Despite the fact that five of the seven ovarian cancers presented as pT3 tumors, only two had lymph node involvement, again indicating distinct features compared to the majority of *BRCA1-* associated ovarian cancers that are mostly poorly differentiated invasive serous adenocarcinomas.

### RAD51C depending cancer susceptibility

In total, six mono-allelic germ-line alterations in *RAD51C* were classified as pathogenic: the two insertions, the two splice site mutations and the two missense alterations G125V and L138F. The corresponding pedigrees are shown in Figure 1A-F. The overall percentage for a pathogenic mutation in *RAD51C* is 0.55% (6/1.100). However, when analyzing the six pedigrees with clearly pathogenic mutations (Figure 1A-F) the inventors observed that all pedigrees presented both, breast and ovarian cancers over different generations, in siblings or even as metachronous tumors. Thus, the six clearly pathogenic *RAD51C* mutations were all found within the 480 BC/OC pedigrees included here (1.3%), suggesting that these mutations confer an increased risk for breast and ovarian cancer.

In these six *RAD51C*-associated BC/OC pedigrees, the mean age of onset at first diagnosis was 53 years (range 33-78) for breast cancer and 60 years (range 50-81) for ovarian cancer. This is higher than the mean age of 40 years and 46 years for BRCA1- and BRCA2-associated breast cancer, respectively, however lower than the mean age of 63 years for sporadic breast cancer. For ovarian cancer, the mean age of onset in *BRCA1* and *BRCA2* mutation carriers and the general population was 49, 58, and 68 years, respectively (German Consortium for Hereditary Breast and Ovarian Cancer, unpublished data, Robert Koch Institute, Berlin, Germany). No occurrence of male breast cancer was noted in these families. In addition, the segregation pattern in these six families is striking: none of the tested healthy females over 70 years of age inherited the mutations (the *unaffected* obligate carriers of the L138F mutation in Figure 1F and of the frameshift mutation c.224_225insA in Figure 1A had surgery with removal of ovaries and uterus due to a lower abdominal tumor at 43 and 40 years of age, respectively) and all of the affected first degree relatives who developed malignancies and could be subjected to mutation analysis turned out to be carriers. This pattern of apparently complete segregation in the *RAD51C* families is clearly different from families carrying *ATM, CHK2, FANCJ*/*BRIP1* and *FANCN*/*PALB2* mutations. In summary, these findings strongly suggest that non-functional mutations in *RAD51C* confer a cancer susceptibility to mutation carriers in the range of *BRCA1* and *BRCA2.*

The germ-line mutations of *RAD51C* in BC/OC families are the first clear link of mutations in *RAD51C* with human disease, showing a high penetrance and causative link of mutations impairing RAD51C function with the occurrence of gynecological cancers. Ultimately, the described mono-allelic germ-line mutations of *RAD51C* are most important to females with a strong family history for breast and ovarian cancer, but negative for mutations in *BRCA1* or *BRCA2.*

**Table 3**

| **Fig.1** | **Site** | **Nucleotide change** | **Protein change** | **Predictive algorithms** | | **Complementation of Δ*Rad51c* DT40 cells** | **Complementation of RAD51C-mutated fibroblasts** | **LOH/ in tumors** | **BC (n=620)** | **BC/OC (n=480)** | **Controls (n=2912)** |
|---|---|---|---|---|---|---|---|---|---|---|---|
| | | | | **SIFT** | **Poly-Phen** | **Survival of cells** | **RAD51 foci** | **(cases)** | | | |
| **A** | **Ex 2** | **c.224_225insA** | **Y75XfsX0** | - | - | - | - | | 0 | 1 | 0 |
| **B** | **Ex 3** | **c.525_526insC** | **C176LfsX26** | - | - | - | - | | 0 | 1 | 0 |
| **C** | **IVS 1** | **c.145+1 G>T** | **V15KfsX9** | - | - | - | - | **1/1** | 0 | 1 | 0 |
| **D** | **IVS 6** | **c.904+5G>T** | **V280GfsX11** | - | - | - | - | **2/2** | 0 | 1 | 0 |
| **E** | **Ex 2** | **c.374 G>T** | **G125V** | yes | Pb | **no** | **no** | **4/4** | 0 | 1 | 0 |
| **F** | **Ex 3** | **c.414 G>C** | **L138F** | yes | Ps | **no** | **reduced** | **3/3** | 0 | 1 | 0 |
| **G** | **Ex 3** | **c.475 G>A** | **D159N** | yes | Ps | **reduced** | **normal** | | 1 | 0 | 0 |
| **H** | **Ex 9** | **c.1097 G>A** | **R366Q** | yes | Ps | **reduced** | **normal** | **0/1** | 0 | 1 | 0 |
| **-** | **Ex 5** | **c.790 G>A** | **G264S** | no | No | **reduced** | **normal** | | 3 | 9^{#} | 16^{#} |
| **-** | **Ex 6** | **c.859 A>G** | **T287A** | no | Pb | **reduced** | **normal** | | 12 | 3 | 35 |
| **-** | **Ex 1** | **c.7 G>A** | **G3R** | yes | Pb | **normal** | **normal** | **0/1** | 0 | 1 | 0* |
| **-** | **Ex 2** | **c.376 G>A** | **A126T** | no | No | **normal** | **normal** | | 13 | 3 | 8* |
| **-** | **Ex 3** | **c.506 T>C** | **V169A** | no | No | **normal** | **normal** | | 1 | 0 | 0* |
| **-** | **Ex 6** | **c.791 G>T** | **G264V** | no | Pb | **normal** | **normal** | | 1 | 0 | 0* |

| | | | | | | | | | | | |
|---|---|---|---|---|---|---|---|---|---|---|---|
| Mutations or variants identified through analyses of individuals with familial breast and/or ovarian cancer. SIFT: yes = affects function; no= tolerated; PolyPhen: ps= possibly damaging; pb = probably damaging; no = benign; # OR for BC/OC = 3.44, Cl = 1.51-7.80, p = 5.32x10⁻³; * n=620 | | | | | | | | | | | |

### References

Arking, D.E. et al. A common genetic variant in the NOS1 regulator NOS1AP modulates cardiac repolarization. Nat Genet 38, 644-51 (2006).
Betz, B. et al. Comparative in silico analyses and experimental validation of novel splice site and missense mutations in the genes MLH1 and MSH2. J Cancer Res Clin Oncol (2009).
Dosanjh, M.K. et al. Isolation and characterization of RAD51C, a new human member of the RAD51 family of related genes. Nucleic Acids Res 26, 1179-84 (1998).
Fong PC, Boss DS, Yap TA, Tutt A, Wu P, Mergui-Roelvink M, Mortimer P, Swaisland H, Lau A, O'Connor MJ, Ashworth A, Carmichael J, Kaye SB, Schellens JH, de Bono JS. Inhibition of poly(ADP-ribose) polymerase in tumors from BRCA mutation carriers. N Engl J Med. Jul 9;361(2):123-34 (2009).
Jones, S. et al. Exomic sequencing identifies PALB2 as a pancreatic cancer susceptibility gene. Science 324, 217 (2009).
Thomas, G. et al. A multistage genome-wide association study in breast cancer identifies two new risk alleles at 1p11.2 and 14q24.1 (RAD51L1). Nat Genet (2009).
Vaz, F. et al. Mutation of the RAD51C gene in Fanconi anemia. Nat Genet re-submitted (2010).
Wilson, J.B. et al. FANCG promotes formation of a newly identified protein complex containing BRCA2, FANCD2 and XRCC3. Oncogene 27(2008).

## Claims

1. An *in vitro* method for determining a predisposition of a subject for developing breast or ovarian cancer comprising the step of analyzing *in vitro* a sample of the subject for an alteration of at least one allele of the RAD51C gene, wherein the alteration of the RAD51C gene leads to an alteration of the RAD51C gene product with reduced or abolished functionality and indicates a predisposition for developing the cancer.

2. The method of claim 1, wherein the alteration of the RAD51C gene is a mutation of the RAD51C gene, preferably a germ-line mutation, further preferred a mono-allelic germ-line mutation.

3. The method of claim 1 or 2, wherein the alteration of the RAD51C gene is a splice site alteration, preferably c.145+1G>T and/or c.904+5G>T, a missense alteration, preferably c.475 G>A, c.1097 G>A, c.374 G>T and/or c.414 G>C, more preferred c.374 G>T and/or c.414 G>C and/or an insertion alteration, preferably c.224_225insA and/or c.525_526insC.

4. The method of any of the preceding claims, wherein the alteration of the RAD51C gene product is an alteration of a codon encoding an amino acid residue of the RAD51C protein.

5. The method of claim 4, wherein the alteration of the codon is selected from the group consisting of Y75XfsX0, C176LfsX26, V15KfsX9, V280GfsX11, G125V, L138F, D159N and R366Q.

6. An *in vitro* method for assessing a pathological progression of a breast or ovarian cancer comprising the step of analyzing *in vitro* a sample of the cancer for a mono-allelic germ-line mutation of the RAD51C gene, wherein the presence of at least one mono-allelic germ-line mutation in the RAD51C gene leads to an alteration of the RAD51C gene product with reduced or abolished functionality and indicates an increased probability for malignancy and/or invasiveness.

7. An *in vitro* method for assessing the functionality of a RAD51C gene product, which is derived from a RAD51C gene comprising at least one alteration, in a cell derived from a subject suffering from breast or ovarian cancer, comprising the steps of
(i) introducing the RAD51C gene into the cell,
(ii) analyzing at least one cellular function, and
(iii) comparing the at least one cellular function to a control cell,
wherein a difference between the cellular function of the cell and the control cell indicates an altered function of the RAD51C gene product and wherein reduced or abolished functionality of the RAD51C gene product indicates a predisposition of the subject for developing breast or ovarian cancer.

8. The method of claim 7, wherein at least one allele of the RAD51C gene (RAD51C +/-), preferably both alleles of the RAD51C gene (RAD51C -/-) is/ are mutated.

9. Use of a kit for determining a predisposition of a subject for developing a cancer by determining the presence of at least one mono-allelic germ-line mutation of the RAD51C gene, wherein the presence of at least one mono-allelic germ-line mutation in the RAD51C gene leads to an alteration of the RAD51C gene product with reduced or abolished functionality and indicates a predisposition for developing cancer, said kit comprising oligonucleotides capable of determining the presence of at least one mono-allelic germ-line mutation of the RAD51C gene.

10. The use of claim 9, wherein the oligonucleotides are selected from the group consisting of SEQ ID NO:11 to SEQ ID NO: 28, in particular a least a combination of SEQ ID NO:11/12, SEQ ID NO: 13/14, SEQ ID NO: 15/16; SEQ ID NO: 17/18, SEQ ID NO: 19/20, SEQ ID NO: 21/22, SEQ ID NO: 23/24, SEQ ID NO: 25/26 and 27/28.

11. Use of oligonucleotides capable of determining the presence of at least one mono-allelic germ-line mutation of the RAD51C gene in a sample of a subject leading to a RAD51C gene product with reduced or abolished functionality for determining a predisposition of the subject for developing breast or ovarian cancer.

12. Use of claim 11, wherein the oligonucleotides are selected from the group consisting of SEQ ID NO:11 to SEQ ID NO: 28, in particular at least a combination of SEQ ID NO-11/12, SEQ ID NO: 13/14, SEQ ID NO: 15/16; SEQ ID NO: 17/18, SEQ ID NO: 19/20, SEQ ID NO: 21/22, SEQ ID NO: 23/24, SEQ ID NO: 25/26 and 27/28.

## Patentansprüche

1. *In vitro* Verfahren zum Bestimmen einer Veranlagung eines Subjekts zum Entwickeln von Brust- oder Eierstockkrebs umfassend den Schritt Analysieren einer Probe des Subjekts *in vitro* auf eine Änderung von mindestens einem Allel des RAD51C-Gens, wobei die Änderung des RAD51C-Gens zu einer Änderung des RAD51C-Genprodukts mit verringerter oder aufgehobener Funktionalität führt und eine Veranlagung zum Entwickeln des Krebses anzeigt.

2. Verfahren nach Anspruch 1, wobei die Änderung des RAD51C-Gens eine Mutation des RAD51C-Gens, vorzugsweise eine Keimbahnmutation, weiter bevorzugt eine mono-allelische Keimbahnmutation ist.

3. Verfahren nach Anspruch 1 oder 2, wobei die Änderung des RAD51C-Gens eine Spleißstellenänderung, vorzugsweise c.145+1G>T und/oder c.904+5G>T, eine Missense-Änderung, vorzugsweise c.475 G>A, c.1097 G>A, c.374 G>T und/oder c.414 G>C, weiter bevorzugt c.374 G>T und/oder c.414 G>C und/oder eine Insertionsänderung, vorzugsweise c.224_225insA und/oder c.525_526insC ist.

4. Verfahren nach einem der vorhergehenden Ansprüche, wobei die Änderung des RAD51C-Gens eine Änderung eines Codons, das einen Aminosäurerest des RAD51C-Proteins kodiert, ist.

5. Verfahren nach Anspruch 4, wobei die Änderung des Codons aus der Gruppe bestehend aus Y75XfsX0, C176LfsX26, V15KfsX9, V280GfsX11, G125V, L138F, D159N und R366Q ausgewählt ist.

6. *In vitro* Verfahren zum Bewerten einer pathologischen Fortschreitung eines Brust- oder Eierstockkrebses umfassend den Schritt Analysieren einer Probe des Krebses *in vitro* auf eine mono-allelische Keimbahnmutation des RAD51C-Gens, wobei die Anwesenheit von mindestens einer mono-allelischen Keimbahnmutation im RAD51C-Gen zu einer Änderung des RAD51C-Genprodukts mit verringerter oder aufgehobener Funktionalität führt und eine erhöhte Wahrscheinlichkeit für Bösartigkeit und/oder Invasivität anzeigt.

7. *In vitro* Verfahren zum Bewerten der Funktionalität eines RAD51C-Genprodukts, welches von einem RAD51C-Gen, das mindestens eine Änderung umfasst, abstammt, in einer Zelle, die aus einem Subjekt, das an Brust- oder Eierstockkrebs leidet, stammt, umfassend die Schritte
(i) Einführen des RAD51C-Gens in die Zelle,
(ii) Analysieren mindestens einer zellulären Funktion, und
(iii) Vergleichen der mindestens einen zellulären Funktion mit einer Kontroll-Zelle,
wobei ein Unterscheid zwischen der zellulären Funktion der Zelle und der Kontrollzelle eine geänderte Funktion des RAD51C-Genprodukts anzeigt und wobei eine verringerte oder aufgehobene Funktionalität des RAD51C-Genprodukts eine Veranlagung des Subjekts zum Entwickeln von Brust- oder Eierstockkrebs anzeigt.

8. Verfahren nach Anspruch 7, wobei mindestens ein Allel des RAD51C-Gens (RAD51C +/-), vorzugsweise beide Allele des RAD51C-Gens (RAD51C -/-) mutiert ist/sind.

9. Verwendung eines Kits zum Bestimmen einer Veranlagung eines Subjekts zum Entwickeln eines Krebses durch Bestimmen der Anwesenheit von mindestens einer mono-allelischen Keimbahnmutation des RAD51C-Gens, wobei die Anwesenheit von mindestens einer mono-allelischen Keimbahnmutation im RAD51C-Gen zu einer Änderung des RAD51C-Genprodukts mit verringerter oder aufgehobener Funktionalität führt und eine Veranlagung zum Entwickeln von Krebs anzeigt, wobei das Kit Oligonukleotide, die in der Lage sind, die Anwesenheit von mindestens einer mono-allelischen Keimbahnmutation des RAD51C-Gens zu bestimmen, umfasst.

10. Verwendung nach Anspruch 9, wobei die Oligonukleotide aus der Gruppe bestehend aus SEQ ID NO: 11 bis SEQ ID NO: 28, insbesondere mindestens einer Kombination von SEQ ID NO: 11/12, SEQ ID NO: 13/14, SEQ ID NO: 15/16; SEQ ID NO: 17/18, SEQ ID NO: 19/20, SEQ ID NO: 21/22, SEQ ID NO: 23/24, SEQ ID NO: 25/26 und 27/28 ausgewählt sind.

11. Verwendung von Oligonukleotiden, die in der Lage sind, die Anwesenheit von mindestens einer mono-allelischen Keimbahnmutation des RAD51C-Gens in einer Probe eines Subjekts zu bestimmen, die zu einem RAD51C-Genprodukt mit verringerter oder aufgehobener Funktionalität führt, zum Bestimmen einer Veranlagung des Subjekts zum Entwickeln von Brust- oder Eierstockkrebs.

12. Verwendung nach Anspruch 11, wobei die Oligonukleotide aus der Gruppe bestehend aus SEQ ID NO: 11 bis SEQ ID NO: 28, insbesondere mindestens einer Kombination von SEQ ID NO: 11/12, SEQ ID NO: 13/14, SEQ ID NO: 15/16; SEQ ID NO: 17/18, SEQ ID NO: 19/20, SEQ ID NO: 21/22, SEQ ID NO: 23/24, SEQ ID NO: 25/26 und 27/28 ausgewählt sind.

## Revendications

1. Procédé *in vitro* destiné à déterminer une prédisposition d'un sujet à développer un cancer du sein ou de l'ovaire comprenant l'étape consistant à analyser *in vitro* un échantillon du sujet pour déceler une altération d'au moins un allèle du gène RAD51C, dans lequel l'altération du gène RAD51C conduit à une altération du produit du gène RAD51C avec une fonctionnalité réduite ou abolie et indique une prédisposition à développer le cancer.

2. Procédé selon la revendication 1, dans lequel l'altération du gène RAD51C est une mutation du gène RAD51C, de préférence une mutation germinale, plus préférablement une mutation germinale monoallélique.

3. Procédé selon la revendication 1 ou 2, dans lequel l'altération du gène RAD51C est une altération de site d'épissage, de préférence c.145+1G>T et/ou c.904+5G>T, une altération faux-sens, de préférence c.475 G>A, c.1097 G>A, c.374 G>T et/ou c.414 G>C, plus préférablement c.374 G>T et/ou c.414 G>C et/ou une altération d'insertion, de préférence c.224_225insA et/ou c.525_526insC.

4. Procédé selon l'une quelconque des revendications précédentes, dans lequel l'altération du produit du gène RAD51C est une altération d'un codon codant pour un résidu d'acide aminé de la protéine RAD51C.

5. Procédé selon la revendication 4, dans lequel l'altération du codon est sélectionnée dans le groupe constitué par Y75XfsX0, C176LfsX26, V15KfsX9, V280GfsX11, G125V, L138F, D159N et R366Q.

6. Procédé *in vitro* destiné à évaluer une progression pathologique d'un cancer du sein ou de l'ovaire comprenant l'étape consistant à analyser *in vitro* un échantillon du cancer pour déceler une mutation germinale monoallélique du gène RAD51C, dans lequel la présence d'au moins une mutation germinale monoallélique dans le gène RAD51C conduit à une altération du produit du gène RAD51C avec une fonctionnalité réduite ou abolie et indique une probabilité accrue de malignité et/ou de caractère invasif.

7. Procédé *in vitro* destiné à évaluer la fonctionnalité d'un produit du gène RAD51C, qui est dérivé d'un gène RAD51C comprenant au moins une altération, dans une cellule dérivée d'un sujet souffrant d'un cancer du sein ou de l'ovaire, comprenant les étapes consistant à
(i) introduire le gène RAD51C dans la cellule,
(ii) analyser au moins une fonction cellulaire, et
(iii) comparer l'au moins une fonction cellulaire à une cellule témoin,
dans lequel une différence entre la fonction cellulaire de la cellule et la cellule témoin indique une fonction altérée du produit du gène RAD51C et dans lequel une fonctionnalité réduite ou abolie du produit du gène RAD51C indique une prédisposition du sujet à développer un cancer du sein ou de l'ovaire.

8. Procédé selon la revendication 7, dans lequel au moins un allèle du gène RAD51C (RAD51C +/-), de préférence les deux allèles du gène RAD51C (RAD51C -/-), est/sont mutés.

9. Utilisation d'un kit destiné à déterminer une prédisposition d'un sujet à développer un cancer en déterminant la présence d'au moins une mutation germinale monoallélique du gène RAD51C, dans laquelle la présence d'au moins une mutation germinale monoallélique dans le gène RAD51C conduit à une altération du produit du gène RAD51C avec une fonctionnalité réduite ou abolie et indique une prédisposition à développer un cancer, ledit kit comprenant des oligonucléotides capables de déterminer la présence d'au moins une mutation germinale monoallélique du gène RAD51C.

10. Utilisation selon la revendication 9, dans laquelle les oligonucléotides sont sélectionnés dans le groupe constitué par les SEQ ID N° : 11 à SEQ ID N° : 28, en particulier au moins une combinaison des SEQ ID N° : 11/12, SEQ ID N° : 13/14, SEQ ID N° : 15/16, SEQ ID N° : 17/18, SEQ ID N° : 19/20, SEQ ID N° : 21/22, SEQ ID N° : 23/24, SEQ ID N° : 25/26 et 27/28.

11. Utilisation d'oligonucléotides capables de déterminer la présence d'au moins une mutation germinale monoallélique du gène RAD51C dans un échantillon d'un sujet conduisant à un produit du gène RAD51C avec une fonctionnalité réduite ou abolie pour déterminer une prédisposition du sujet à développer un cancer du sein ou de l'ovaire.

12. Utilisation selon la revendication 11, dans laquelle les oligonucléotides sont sélectionnés dans le groupe constitué par les SEQ ID N° : 11 à SEQ ID N° : 28, en particulier au moins une combinaison des SEQ ID N° : 11/12, SEQ ID N° : 13/14, SEQ ID N° : 15/16, SEQ ID N° : 17/18, SEQ ID N° : 19/20, SEQ ID N° : 21/22, SEQ ID N° : 23/24, SEQ ID N° : 25/26 et 27/28.


### SEQUENCE LISTING

<110> Hanenberg, Helmut Niederacher, Dieter
<120> RAD51C as a human cancer susceptibility gene
<130> H30007
<160> 28
<170> PatentIn version 3.3
<210> 1
   <211> 263
   <212> DNA
   <213> Homo sapiens
<400> 1
   acgccgcacg ccccagcgag ggcgtgcgga gtttggctgc tccggggtta gcaggtgagc     60
   ctgcgatgcg cgggaagacg ttccgctttg aaatgcagcg ggatttggtg agtttcccgc    120
   tgtctccagc ggtgcgggtg aagctggtgt ctgcggggtt ccagactgct gaggaactcc    180
   tagaggtgaa accctccgag cttagcaaag gtaacgactc ctgatggcaa gctgaggcac    240
   accggccgcc gtcagcgccg cct                                            263
<210> 2
   <211> 408
   <212> DNA
   <213> Homo sapiens
<400> 2
   tctacaaatt aataaagaca atcgattatc atgttacact tttaaatctc taaaattagg    60
   gttctttttt tcttatttta ctttcagaag ttgggatatc taaagcagaa gccttagaaa   120
   ctctgcaaat tatcagaaga gaatgtctca caaataaacc aagatatgct ggtacatctg   180
   agtcacacaa gaagtgtaca gcactggaac ttcttgagca ggagcatacc cagggcttca   240
   taatcacctt ctgttcagca ctagatgata ttcttggggg tggagtgccc ttaatgaaaa   300
   caacagaaat ttgtggtgca ccaggtgttg gaaaaacaca attatggtaa aataaagtgt   360
   tctcctttta agggtgggtt taataacata ttatgaaagt agtatttt                408
<210> 3
   <211> 348
   <212> DNA
   <213> Homo sapiens
<400> 3
<210> 4
   <211> 281
   <212> DNA
   <213> Homo sapiens
<400> 4
   aaaactaatt aagagtgttt tgttgtttca gaacaccgaa aagctttgga ggatttcact    60
   cttgataata ttctttctca tatttattat tttcgctgtc gtgactacac agagttactg   120
   gcacaagttt atcttcttcc agatttcctt tcagaacact caaaggtatg agtcagacta   180
   ctgaaatgta actaaccaag tattttttga ggtgtttgat aagcatgaaa aaataaccag   240
   tacagtagca taaaatcaaa gtcaaagcca attgagaaaa t                       281
<210> 5
   <211> 509
   <212> DNA
   <213> Homo sapiens
<400> 5
   tttttccaat gctatgtttt tttctatcta gtaagggttg gattaaagaa gaggctttta    60
   tgaagcaatg tctaagtaag ttgttttatt tagagtattt gtttcttcat ttagcaagta   120
   ttaattgaca cctcctttcc tatatgctat ttactgttcc aggcattggg gatgatatag   180
   taaataagac agaagaatat agtaaataag agagaaggtc cctgctctct tggagagaga   240
   gagcattttt attattatta ttttattttt cgtaacaaat ctaatattat ctcttctgta   300
   tttaggttcg actagtgata gtggatggta ttgcttttcc atttcgtcat gacctagatg   360
   acctgtctct tcgtactcgg ttattaaatg gcctagccca gcaaatgatc agccttgcaa   420
   ataatcacag attagctgta agtattaact agtgaagaga gttttataac aaagtcaaga   480
   ctgtataaaa tgttaatgtc tagaaatgt                                     509
<210> 6
   <211> 250
   <212> DNA
   <213> Homo sapiens
<400> 6
   tacttgataa ttttcaaaga gactcaccta attttcttac attttgtttt tgtaggtaat    60
   tttaaccaat cagatgacaa caaagattga tagaaatcag gccttgcttg ttcctgcatt   120
   aggtgggtaa ttaatcagat aaacatttta gtttatcaca gtttttctta tctctttcat   180
   ttgattctca ttgagtacta tacgcttcat gaaagcagac tgtatttgtc ttgttcactg   240
   gttaatctta                                                          250
<210> 7
   <211> 303
   <212> DNA
   <213> Homo sapiens
<400> 7
   tatactttcg ttatgttaaa ttaataaagt aagattatat ttgatcagag gcgttctgag    60
   aaatgtataa ccaagtcagt aaggccatat acagttatta tgttttttac tctcagggga   120
   aagttgggga catgctgcta caatacggct aatctttcat tgggaccgaa agcaaaggtc   180
   agtacagaaa caagttaata actccgaata ttgggttaat tatactgaat gaacacttac   240
   aggtttctta gagctagtcc tgtggatgag atatacagtg acccatgaag tgacactttt   300
   gtt                                                                 303
<210> 8
   <211> 303
   <212> DNA
   <213> Homo sapiens
<400> 8
   tgtattttta atatttctct cctttttgtg ttcttagaga aaaaatagaa ttattaatat     60
   aataaaccta tacatttaaa taatgagttt ggtcatctga acttttaatt aattaagttc   120
   atgtgtttgt atgtatttat tctttttctt taagcaggtt ggcaacattg tacaagtcac   180
   ccagccagaa ggaatgcaca gtactgtttc aaatcaaagt cagtattatt tgattagagt   240
   gggattttga tattgatggg cggtaattat ctaaagagag aatttacaac ttgcttctgt   300
   caa                                                                 303
<210> 9
   <211> 246
   <212> DNA
   <213> Homo sapiens
<400> 9
   ctttcttatt agttacttaa aaatatttct aagatcagtc ttcaaatgtt cttaaagcat    60
   atttgtatat atatttttta tctttcagcc tcagggattt agagatactg ttgttacttc   120
   tgcatgttca ttgcaaacag aaggttcctt gagcacccgg aaacggtcac gagacccaga   180
   ggaagaatta taacccagaa acaaatctca aagtgtacaa atttattgat gttgtgaaat   240
   caatgt                                                              246
<210> 10
   <211> 1131
   <212> DNA
   <213> Homo sapiens
<400> 10
   atgcgcggga agacgttccg ctttgaaatg cagcgggatt tggtgagttt cccgctgtct    60
   ccagcggtgc gggtgaagct ggtgtctgcg gggttccaga ctgctgagga actcctagag   120
   gtgaaaccct ccgagcttag caaagaagtt gggatatcta aagcagaagc cttagaaact   180
   ctgcaaatta tcagaagaga atgtctcaca aataaaccaa gatatgctgg tacatctgag   240
   tcacacaaga agtgtacagc actggaactt cttgagcagg agcataccca gggcttcata   300
   atcaccttct gttcagcact agatgatatt cttgggggtg gagtgccctt aatgaaaaca   360
   acagaaattt gtggtgcacc aggtgttgga aaaacacaat tatgtatgca gttggcagta   420
   gatgtgcaga taccagaatg ttttggagga gtggcaggtg aagcagtttt tattgataca 480
   gagggaagtt ttatggttga tagagtggta gaccttgcta ctgcctgcat tcagcacctt 540
   cagcttatag cagaaaaaca caagggagag gaacaccgaa aagctttgga ggatttcact 600
   cttgataata ttctttctca tatttattat tttcgctgtc gtgactacac agagttactg 660
   gcacaagttt atcttcttcc agatttcctt tcagaacact caaaggttcg actagtgata 720
   gtggatggta ttgcttttcc atttcgtcat gacctagatg acctgtctct tcgtactcgg 780
   ttattaaatg gcctagccca gcaaatgatc agccttgcaa ataatcacag attagctgta 840
   attttaacca atcagatgac aacaaagatt gatagaaatc aggccttgct tgttcctgca 900
   ttaggggaaa gttggggaca tgctgctaca atacggctaa tctttcattg ggaccgaaag 960
   caaaggttgg caacattgta caagtcaccc agccagaagg aatgcacagt actgtttcaa 1020
   atcaaacctc agggatttag agatactgtt gttacttctg catgttcatt gcaaacagaa 1080
   ggttccttga gcacccggaa acggtcacga gacccagagg aagaattata a 1131
<210> 11
   <211> 20
   <212> DNA
   <213> Artificial
<220>
   <223> forward primer Ex1
<400> 11
   tccgctttac gtctgacgtc 20
<210> 12
   <211> 20
   <212> DNA
   <213> Artificial
<220>
   <223> reverse primer Ex1
<400> 12
   aggcgagaga acgaagactg 20
<210> 13
   <211> 20
   <212> DNA
   <213> Artificial
<220>
   <223> forward primer Ex2
<400> 13
   cactcctagc atcactgttg 20
<210> 14
   <211> 21
   <212> DNA
   <213> Artificial
<220>
   <223> reverse primer Ex2
<400> 14
   ttggtttcct gacgatagta c 21
<210> 15
   <211> 20
   <212> DNA
   <213> Artificial
<220>
   <223> forward primer Ex3
<400> 15
   atttctgttg ccttggggag 20
<210> 16
   <211> 20
   <212> DNA
   <213> Artificial
<220>
   <223> reverse primer Ex3
<400> 16
   aatggagtgt tgctgaggtc 20
<210> 17
   <211> 21
   <212> DNA
   <213> Artificial
<220>
   <223> forward primer Ex4
<400> 17
   tgccaataca tccaaacagg t 21
<210> 18
   <211> 20
   <212> DNA
   <213> Artificial
<220>
   <223> reverse primer Ex4
<400> 18
   gtaggtcaag gaaggaagag 20
<210> 19
   <211> 21
   <212> DNA
   <213> Artificial
<220>
   <223> forward primer Ex5
<400> 19
   ttttcctgta atggactatg g 21
<210> 20
   <211> 21
   <212> DNA
   <213> Artificial
<220>
   <223> reverse primer Ex5
<400> 20
   tgtcaggcaa acgctatttt g 21
<210> 21
   <211> 23
   <212> DNA
   <213> Artificial
<220>
   <223> forward primer Ex6
<400> 21
   tcacaatctt ggccagactg gtc 23
<210> 22
   <211> 20
   <212> DNA
   <213> Artificial
<220>
   <223> reverse primer Ex6
<400> 22
   aacggtactg tgcttagtgc 20
<210> 23
   <211> 22
   <212> DNA
   <213> Artificial
<220>
   <223> forward primer Ex7
<400> 23
   ttccaggttt tttgaaagca ag 22
<210> 24
   <211> 20
   <212> DNA
   <213> Artificial
<220>
   <223> reverse primer Ex7
<400> 24
   taggtgatat cagacaaggc 20
<210> 25
   <211> 20
   <212> DNA
   <213> Artificial
<220>
   <223> forward primer Ex8
<400> 25
   catacgggta atttgaaggg 20
<210> 26
   <211> 20
   <212> DNA
   <213> Artificial
<220>
   <223> reverse primer Ex8
<400> 26
   atgcttgctg cctacagaag 20
<210> 27
   <211> 20
   <212> DNA
   <213> Artificial
<220>
   <223> forward primer Ex9
<400> 27
   ctggccctag aataaagtag 20
<210> 28
   <211> 20
   <212> DNA
   <213> Artificial
<220>
   <223> reverse primer Ex9
<400> 28
   gtaacaagt ccacttgtac 20